# EUROPEAN PATENT APPLICATION

(11) **EP 4 830 750 A2**
(43) Date of publication of application: **28.10.2026**
(21) Application number: 26170950.5
(22) Date of filing: 25.02.2022
(51) Int. Cl.: A61M 5/48

(54) **AIR DETECTION AND MEASUREMENT SYSTEM FOR FLUID INJECTOR**

(30) Priority: 26.02.2021 US 202163154184 P
(62) Document number(s) of the earlier application(s) in accordance with Art. 76 EPC: 22710837.0 / 4 297 818
(71) Applicant: Bayer Healthcare LLC, Whippany, NJ 07981 (US)
(72) Inventor: Barone, William, Pittsburgh, Pennsylvania, 15217 (US); Brown, Ronald, West Newton, Pennsylvania, 15089 (US); Pastor, Curtis, Allen, Texas, 75013 (US); Capone, Christopher, Pittsburgh, Pennsylvania, 15238 (US); Wlodarczyk, Jaroslaw, Lower Burrell, Pennsylvania, 15068 (US); Lang, Charles, Pittsburgh, Pennsylvania, 15237 (US); Swantner, Michael, Saxonburg, Pennsylvania, 16056 (US)
(74) Representative: BIP Patents

(57) **Abstract**

A fluid injector system includes at least one injector for pressurizing and delivering at least one fluid from at least one fluid reservoir, at least one fluid path section in fluid communication with the at least one injector and having a predetermined index of refraction, and a first proximal sensor and a first distal sensor arranged along the at least one fluid path section. Each of the first proximal sensor and the first distal sensor includes an emitter configured to emit light through the at least one fluid path section, and a detector configured to receive the light emitted through the at least one fluid path section and generate an electrical signal based on the received light. The fluid injector system further includes at least one processor programmed or configured to determine, based on a difference in the electrical signals generated by the first proximal sensor and the first distal sensor, at least one property of a content of the at least one fluid path section.

## Description

### CROSS-REFERENCE TO RELATED APPLICATION

The present application claims the benefit of United States Provisional Patent Application No. 63/154,184, filed February 26, 2021, the disclosure of which is hereby incorporated by reference in its entirety.

### BACKGROUND OF THE DISCLOSURE

### Field of the Disclosure

The present disclosure relates generally to fluid path configurations and apparatuses for use with fluid injectors for pressurized injection of medical fluids. Specifically, the present disclosure describes systems, fluid path sets, and methods for detection and measurement of air in the fluid flow to address inadvertent air injection during an injection procedure.

### Description of Related Art

**In** many medical diagnostic and therapeutic procedures, a medical practitioner injects a patient with one or more medical fluids. In recent years, a number of injector-actuated syringes and powered fluid injectors for pressurized injection of medical fluids, such as an imaging contrast media solution (often referred to simply as "contrast"), a flushing agent, such as saline or Ringer's lactate, and other medical fluids, have been developed for use in imaging procedures such as cardiovascular angiography (CV), computed tomography (CT), ultrasound, magnetic resonance imaging (MRI), positron emission tomography (PET), and other imaging procedures. In general, these fluid injectors are designed to deliver a preset amount of fluid at a preset pressure and/or flow rate.

Typically, fluid injectors have at least one drive member, such as a piston, that connects to the syringe, for example via connection with a plunger or an engagement feature on a proximal end wall of the syringe. Alternatively, the fluid injector may include one or more peristaltic pumps for injecting the medical fluid from a fluid reservoir. The syringe may include a rigid barrel with a syringe plunger slidably disposed within the barrel. The drive members drive the plungers in a proximal and/or distal direction relative to a longitudinal axis of the barrel to draw fluid into or deliver the fluid from the syringe barrel, respectively. In certain applications, the medical fluids are injected into the vascular system at fluid pressures up to 300 psi for CT imaging procedures or up to 1200 psi for example for CV imaging procedures.

During certain injection procedures at these high fluid pressures with fluid being administered to the vascular system, it is important that any air or other gas co-injected with the medical fluid to a patient is minimized or eliminated as significant patient harm may result. Thus, new methods and devices are necessary to detect and measure amounts of air flowing toward the patient during an injection procedure and, if the amount of air is greater than a safe threshold, to stop the injection to allow the air to be removed from the injection system.

### SUMMARY OF THE DISCLOSURE

In view of the above-identified needs, the present disclosure provides systems, devices, system components, and methods for detecting and measuring a volume of air present in a fluid line during a medical fluid injection procedure. In certain embodiments, the present disclosure is directed to a fluid injector system, including at least one injector for pressurizing and delivering at least one fluid from at least one fluid reservoir, at least one fluid path section in fluid communication with the at least one injector and having a predetermined index of refraction, and a first proximal sensor and a first distal sensor arranged along the at least one fluid path section. Each of the first proximal sensor and the first distal sensor include an emitter configured to emit light through the at least one fluid path section, and a detector configured to receive the light emitted through the at least one fluid path section and generate an electrical signal based on the received light. The fluid injector system further comprises at least one processor programmed or configured to determine, based on a difference in the electrical signals generated by the first proximal sensor and the first distal sensor, at least one property of a content of the at least one fluid path section.

In some embodiments, the at least one property of the content is selected from at least one of: an identity of the fluid in the fluid path section, the presence of one or more air bubbles in the fluid path section, a volume of one or more air bubbles in the fluid path section, a velocity of one or more air bubbles in the fluid path section, a priming status of the fluid path section, and combinations of any thereof.

In some embodiments, the at least one processor is programmed or configured to determine a velocity of an air bubble passing through the at least one fluid path section based on a time offset between detection of the air bubble by the first proximal sensor and detection of the air bubble by the first distal sensor.

In some embodiments, the emitter of the first proximal sensor is arranged on a first side of the fluid path section, emitter of the first distal sensor is arranged on a second side of the fluid path section, and the second side of the fluid path section is approximately 180° opposite the first side of the fluid path section.

In some embodiments, the controller is configured to actuate the emitter of the first proximal sensor and the emitter of the first distal sensor in alternating pulses.

In some embodiments, the fluid injector system includes a first fluid reservoir and a second fluid reservoir for delivering a first fluid and a second fluid, respectively. The fluid injector system further includes a first fluid path section in fluid communication with the first fluid reservoir and a second fluid path section in fluid communication with the second fluid reservoir, and first and second proximal sensors and first and second distal sensors. The first fluid path section is associated with the first proximal sensor and first distal sensor and the second fluid path section is associated with the second proximal sensor and second distal sensor.

In some embodiments, the fluid injector system further includes a manifold including the first fluid path section and the second fluid path section. The manifold positions the first fluid path section and the second fluid path section to interface with the first and second proximal sensors and the first and second distal sensors, respectively.

In some embodiments, fluid injector system includes a manifold housing module for removably receiving the manifold. The manifold housing module includes the first and second proximal sensors and the first and second distal sensors.

In some embodiments, the manifold includes at least one rib for indexing the manifold within the manifold housing module.

In some embodiments, the emitter and the detector of each of the first and second proximal sensors and the first and second distal sensors are located behind associated optical surfaces of the manifold housing module, and the at least one rib prevents the manifold from contacting the associated optical surfaces of the manifold housing module.

In some embodiments, the manifold housing module includes at least one filter for filtering light from entering the detector.

In some embodiments, at least one of the manifold and the manifold housing module includes a lens for concentrating or dispersing light emitted from the emitter.

In some embodiments, the manifold housing module includes a collimator for collimating light emitted from the emitter.

In some embodiments, the at least one fluid reservoir includes at least one syringe, and the fluid injector system further includes a syringe tip including the at least one fluid path section.

In some embodiments, the fluid injector system further includes a reference detector for receiving light from the emitter that has not passed through the at least one fluid path section.

In some embodiments, the emitter of at least one of the first proximal sensor and the first distal sensor is arranged to emit light perpendicular to a fluid flow direction through the at least one fluid path section.

In some embodiments, the emitter of at least one of the first proximal sensor and the first distal sensor is arranged to emit light at an angle of between approximately 30° and approximately 60° relative to a fluid flow direction through the at least one fluid path section.

In some embodiments, the at least one processor is programmed or configured to halt actuation of the at least one injector in response to determining that the at least one fluid path section contains one or more air bubbles having a total air volume above a predetermined volume.

In some embodiments, the at least one processor is programmed or configured to determine, based on the electrical signal, that the at least one fluid path section is present between the emitter and the detector of each of the first proximal sensor and first distal sensor.

In some embodiments, the emitter of at least one of the first proximal sensor and the first distal sensor is configured to emit light on the ultraviolet spectrum.

In some embodiments, the emitter of at least one of the first proximal sensor and the first distal sensor is configured to emit light on the infrared spectrum.

In some embodiments, the emitter of at least one of the first proximal sensor and the first distal sensor is configured to emit light on the visible spectrum.

In some embodiments, an index of refraction of a sidewall of the at least one fluid path section is closer to an index of refraction of a contrast media than to an index of refraction of air.

Other embodiments of the present disclosure are directed to a fluid manifold for a fluid path component. The fluid manifold includes at least one inlet port configured for fluid communication to at least one fluid reservoir, at least one outlet port configured for fluid communication to at least one administration line, at least one fill port configured for fluid communication to at least one bulk fluid source, and at least one fluid path section in fluid communication with the at least one inlet port, the at least one outlet port, and the at least one fill port. The at least one fluid path section has a sidewall having a predetermined index of refraction such that light passes through the fluid path section at a known refraction.

In some embodiments, the index of refraction of the sidewall of the at least one fluid path section is closer to an index of refraction of water than to an index of refraction of air.

In some embodiments, the at least one fluid path section is rigid.

In some embodiments, the at least one fluid path section includes at least one rib extending radially outward and configured to engage a manifold housing module to index the fluid path section in the manifold housing module.

In some embodiments, the at least one fluid path section has a surface finish configured to concentrate or disperse light passing through the fluid path section.

In some embodiments, one of the manifold housing module and the at least one fluid path section includes at least one lens to concentrate or disperse light passing through the fluid path section.

In some embodiments, the at least one fluid path section is transparent to at least one of ultraviolet light, visible light, and infrared light.

In some embodiments, each of the at least one outlet ports includes a check valve.

In some embodiments, the manifold further includes a first manifold section defining a first fluid path for a first medical fluid, a second manifold section defining a second fluid path for a second medical fluid, and at least one connecting beam connecting the first manifold section to the second manifold section. The first fluid path is isolated from the second fluid path, and the at least one connecting beam orients the first manifold section and the second manifold section in a position to fit within the manifold housing module and correctly interface the first fluid path with a first proximal sensor and a first distal sensor and interface the second fluid path within a second proximal sensor and a second distal sensor.

Other embodiments of the present disclosure are directed to a method for determining one or more fluid properties of a fluid flowing in at least one fluid path section of a fluid injector system. The method includes emitting light from an emitter of a first proximal sensor through a proximal portion of the at least one fluid path section, detecting with a detector of the first proximal sensor the light that has passed through the proximal portion of the at least one fluid path section, emitting light from an emitter of a first distal sensor through a distal portion of the at least one fluid path section, detecting with a detector of the first distal sensor the light that has passed through the distal portion of the at least one fluid path section, and determining at least one property of the fluid as it flows through at least one fluid path section based on a difference in light measurement valves determined by the first proximal sensor and the first distal sensor, the at least one fluid path section has a predetermined index of refraction such that the light passes through the fluid path section at a known refraction.

In some embodiments, the method further includes determining the at least one property of the fluid includes determining whether the at least one fluid path section contains a medical fluid, air, or one or more air bubbles.

In some embodiments, the method further includes determining a velocity of an air bubble passing through the fluid path section based on a time offset between detection of the air bubble by the first proximal sensor and detection of the bubble by the first distal sensor.

In some embodiments, the method further includes determining a volume of an air bubble passing through the fluid path section based on a time offset between detection of a bubble front and a bubble end of the air bubble by the first proximal sensor and detection of the bubble front and the bubble end of the air bubble by the first distal sensor and a pressure of the fluid within the fluid path section.

In some embodiments, the first proximal sensor is arranged on a first side of the fluid path section, the second distal sensor is arranged on a second side of the fluid path section, and the second side of the fluid path section is approximately 180° opposite the first side of the fluid path section.

In some embodiments, the method further includes emitting light from the first proximal sensor and emitting light from the first distal sensor in alternating pulses.

In some embodiments, the fluid injector system includes a first fluid reservoir and a second fluid reservoir for delivering a first fluid and a second fluid, respectively, a first fluid path section in fluid communication with the first fluid reservoir and a second fluid path section in fluid communication with the second fluid reservoir, and first and second proximal sensors and first and second distal sensors. The first fluid path section is associated with the first proximal sensor and the first distal sensor and the second fluid path section is associated with the second proximal sensor and the second distal sensor.

In some embodiments, the method further includes inserting a manifold including the first fluid path section and the second fluid path section into a manifold housing module. The manifold housing module includes the first and second proximal sensors and the first and second distal sensors, and the manifold positions the first fluid path section and the second fluid path section to interface with the first and second proximal sensors and the first and second distal sensors, respectively.

In some embodiments, the manifold includes at least one rib for indexing the manifold within the manifold housing module.

In some embodiments, the emitter and the detector of each of the first proximal sensor and the first distal sensor are located behind associated optical surfaces of the manifold housing module, and the at least one rib prevents the manifold from contacting the associated optical surfaces of the manifold housing module.

In some embodiments, the manifold housing module includes at least one filter for filtering light emitted from the first proximal sensor and the first distal sensor.

In some embodiments, at least one of the manifold and the manifold housing module includes a lens for concentrating or dispersing light emitted from the first proximal sensor and the first distal sensor.

In some embodiments, the manifold housing module includes a collimator for collimating light emitted from the first proximal sensor and the first distal sensor.

In some embodiments, the method further includes detecting, with a reference detector of the first proximal sensor or the first distal sensor, a reference light that has not passed through the at least one fluid path section, and comparing the reference light to the light that has passed through the at least one fluid path section to determine fluid content of the at least one fluid path section.

In some embodiments, the emitter of at least one of the first proximal sensor and the first distal sensor is arranged to emit light perpendicular to a fluid flow direction through the at least one fluid path section.

In some embodiments, the emitter of at least one of the first proximal sensor and the first distal sensor is arranged to emit light at an angle of between approximately 30° and approximately 60° relative to a fluid flow direction through the at least one fluid path section.

In some embodiments, the method further includes halting an injection procedure of the fluid injector system in response to determining that the at least one fluid path section contains one or more air bubbles having a total air volume above a predetermined volume.

In some embodiments, the method further includes determining, based on the detected light, that the at least one fluid path section is present between the emitter and the detector of each of the first proximal sensor and the first distal sensor.

In some embodiments, the emitter of at least one of the first proximal sensor and the first distal sensor is configured to emit light on the ultraviolet spectrum.

In some embodiments, the emitter of at least one of the first proximal sensor and the first distal sensor is configured to emit light on the infrared spectrum.

In some embodiments, the emitter of at least one of the first proximal sensor and the first distal sensor is configured to emit light on the visible spectrum.

In some embodiments, an index of refraction of a sidewall of the at least one fluid path section is closer to an index of refraction of water than to an index of refraction of air.

In some embodiments, the method further includes determining a cumulative total volume of air passing through the at least one fluid path section during an injection procedure by adding the volume of the air bubble to a previous cumulative total volume of air.

Further aspects or examples of the present disclosure are described in the following numbered clauses:
Clause 1. A fluid injector system, comprising: at least one injector for pressurizing and delivering at least one fluid from at least one fluid reservoir; at least one fluid path section in fluid communication with the at least one injector and having a predetermined index of refraction; a first proximal sensor and a first distal sensor arranged along the at least one fluid path section, each of the first proximal sensor and the first distal sensor comprising: an emitter configured to emit light through the at least one fluid path section; and a detector configured to receive the light emitted through the at least one fluid path section and generate an electrical signal based on the received light; and at least one processor programmed or configured to determine, based on a difference in the electrical signals generated by the first proximal sensor and the first distal sensor, at least one property of a content of the at least one fluid path section.
Clause 2. The fluid injector system of clause 1, wherein the at least one property of the content is selected from at least one of an identity of the fluid in the fluid path section, the presence of one or more air bubbles in the fluid path section, a volume of one or more air bubbles in the fluid path section, a velocity of one or more air bubbles in the fluid path section, a priming status of the fluid path section, and combinations of any thereof.
Clause 3. The fluid injector system of clause 1 or 2, wherein the at least one processor is programmed or configured to: determine a velocity of an air bubble passing through the at least one fluid path section based on a time offset between detection of the air bubble by the first proximal sensor and detection of the air bubble by the first distal sensor.
Clause 4. The fluid injector system of any of clauses 1 to 3, wherein the emitter of the first proximal sensor is arranged on a first side of the fluid path section, wherein the emitter of the first distal sensor is arranged on a second side of the fluid path section, and wherein the second side of the fluid path section is approximately 180° opposite the first side of the fluid path section.
Clause 5. The fluid injector system of any of clauses 1 to 4, wherein the controller is configured to actuate the emitter of the first proximal sensor and the emitter of the first distal sensor in alternating pulses.
Clause 6. The fluid injector system of any of clauses 1 to 5, wherein the fluid injector system comprises a first fluid reservoir and a second fluid reservoir for delivering a first fluid and a second fluid, respectively; a first fluid path section in fluid communication with the first fluid reservoir and a second fluid path section in fluid communication with the second fluid reservoir; and first and second proximal sensors and first and second distal sensors, wherein the first fluid path section is associated with the first proximal sensor and the first distal sensor and the second fluid path section is associated with the second proximal sensor and the second distal sensor.
Clause 7. The fluid injector system of any of clauses 1 to 6, wherein the fluid injector system further comprises a manifold comprising the first fluid path section and the second fluid path section, wherein the manifold positions the first fluid path section and the second fluid path section to interface with the first and second proximal sensors and the first and second distal sensors, respectively.
Clause 8. The fluid injector system of any of clauses 1 to 7, further comprising a manifold housing module for removably receiving the manifold, wherein the manifold housing module comprises the first and second proximal sensors and the first and second distal sensors.
Clause 9. The fluid injector system of any of clauses 1 to 8, wherein the manifold comprises at least one rib for indexing the manifold within the manifold housing module.
Clause 10. The fluid injector system of any of clauses 1 to 9, wherein the emitter and the detector of each of the first and second proximal sensors and the first and second distal sensors are located behind associated optical surfaces of the manifold housing module, and wherein the at least one rib prevents the manifold from contacting the associated optical surfaces of the manifold housing module.
Clause 11. The fluid injector system of any of clauses 1-10, wherein the manifold housing module comprises at least one filter for filtering light from entering the detector.
Clause 12. The fluid injector system of any of clauses 1-11, wherein at least one of the manifold and the manifold housing module comprises a lens for concentrating or dispersing light emitted from the emitter.
Clause 13. The fluid injector system of any of clauses 1-12, wherein the manifold housing module comprises a collimator for collimating light emitted from the emitter.
Clause 14. The fluid injector system of any of clauses 1-13, wherein the at least one fluid reservoir comprises at least one syringe, and wherein the fluid injector system further comprises a syringe tip comprising the at least one fluid path section.
Clause 15. The fluid injector system of any of clauses 1-14, further comprising a reference detector for receiving light from the emitter that has not passed through the at least one fluid path section.
Clause 16. The fluid injector system of any of clauses 1-15, wherein the emitter of at least one of the first proximal sensor and the first distal sensor is arranged to emit light perpendicular to a fluid flow direction through the at least one fluid path section.
Clause 17. The fluid injector system of any of clauses 1-16, wherein the emitter of at least one of the first proximal sensor and the first distal sensor is arranged to emit light at an angle of between approximately 30° and approximately 60° relative to a fluid flow direction through the at least one fluid path section.
Clause 18. The fluid injector system any of clauses 1-17, wherein the at least one processor is programmed or configured to halt actuation of the at least one injector in response to determining that the at least one fluid path section contains one or more air bubbles having a total air volume above a predetermined volume.
Clause 19. The fluid injector system of any of clauses 1-18, wherein the at least one processor is programmed or configured to determine, based on the electrical signal, that the at least one fluid path section is present between the emitter and the detector of each of the first proximal sensor and the first distal sensor.
Clause 20. The fluid injector system of any of clauses 1-19, wherein the emitter of at least one of the first proximal sensor and the first distal sensor is configured to emit light on the ultraviolet spectrum.
Clause 21. The fluid injector system of any of clauses 1-20, wherein the emitter of at least one of the first proximal sensor and the first distal sensor is configured to emit light on the infrared spectrum.
Clause 22. The fluid injector system of any of clauses 1-21, wherein the emitter of at least one of the first proximal sensor and the first distal sensor is configured to emit light on the visible spectrum.
Clause 23. The fluid injector system of any of clauses 1-22, wherein an index of refraction of a sidewall of the at least one fluid path section is closer to an index of refraction of a contrast media than to an index of refraction of air.
Clause 24. A fluid manifold for a fluid path component, the fluid manifold comprising: at least one inlet port configured for fluid communication to at least one fluid reservoir; at least one outlet port configured for fluid communication to at least one administration line; at least one fill port configured for fluid communication to at least one bulk fluid source; and at least one fluid path section in fluid communication with the at least one inlet port, the at least one outlet port, and the at least one fill port, the at least one fluid path section having a sidewall having a predetermined index of refraction such that light passes through the fluid path section at a known refraction.
Clause 25. The fluid manifold of clause 24, wherein the index of refraction of the sidewall of the at least one fluid path section is closer to an index of refraction of water than to an index of refraction of air.
Clause 26. The fluid manifold of clause 24 or 25, wherein the at least one fluid path section is rigid.
Clause 27. The fluid manifold of any of clauses 24-26, wherein the at least one fluid path section comprises at least one rib extending radially outward and configured to engage a manifold housing module to index the fluid path section in the manifold housing module.
Clause 28. The fluid manifold of any of clauses 24-27, wherein the at least one fluid path section has a surface finish configured to concentrate or disperse light passing through the fluid path section.
Clause 29. The fluid manifold of any of clauses 24 to 28, wherein one of the manifold housing module and the at least one fluid path section comprises at least one lens to concentrate or disperse light passing through the fluid path section.
Clause 30. The fluid manifold of any of clauses 24-29, wherein the at least one fluid path section is transparent to at least one of ultraviolet light, visible light, and infrared light.
Clause 31. The fluid manifold of any of clauses 24-30, wherein each of the at least one outlet ports comprises a check valve.
Clause 32. The fluid manifold of any of clauses 24-31, further comprising: a first manifold section defining a first fluid path for a first medical fluid; a second manifold section defining a second fluid path for a second medical fluid; and at least one connecting beam connecting the first manifold section to the second manifold section, wherein the first fluid path is isolated from the second fluid path, and wherein the at least one connecting beam orients the first manifold section and the second manifold section in a position to fit within the manifold housing module and correctly interface the first fluid path with a first proximal sensor and a first distal sensor and interface the second fluid path within a second proximal sensor and a second distal sensor.
Clause 33. A method for determining one or more fluid properties of a fluid flowing in at least one fluid path section of a fluid injector system, the method comprising: emitting light from an emitter of a first proximal sensor through a proximal portion of the at least one fluid path section; detecting with a detector of the first proximal sensor the light that has passed through the proximal portion of the at least one fluid path section; emitting light from an emitter of a first distal sensor through a distal portion of the at least one fluid path section; detecting with a detector of the first distal sensor the light that has passed through the distal portion of the at least one fluid path section; and determining at least one property of the fluid as it flows through at least one fluid path section based on a difference in light measurement valves determined by the first proximal sensor and the first distal sensor, wherein the at least one fluid path section has a predetermined index of refraction such that the light passes through the fluid path section at a known refraction.
Clause 34. The method of clause 33, wherein determining the at least one property of the fluid comprises determining whether the at least one fluid path section contains a medical fluid, air, or one or more air bubbles.
Clause 35. The method of clause 33 or 34, further comprising: determining a velocity of an air bubble passing through the fluid path section based on a time offset between detection of the air bubble by the first proximal sensor and detection of the bubble by the first distal sensor.
Clause 36. The method of any of clauses 33-35, further comprising: determining a volume of an air bubble passing through the fluid path section based on a time offset between detection of a bubble front and a bubble end of the air bubble by the first proximal sensor and detection of the bubble front and the bubble end of the air bubble by the first distal sensor and a pressure of the fluid within the fluid path section.
Clause 37. The method of any of clauses 33-36, wherein the first proximal sensor is arranged on a first side of the fluid path section, wherein the second distal sensor is arranged on a second side of the fluid path section, and wherein the second side of the fluid path section is approximately 180° opposite the first side of the fluid path section.
Clause 38. The method of any of clauses 33-37, further comprising emitting light from the first proximal sensor and emitting light from the first distal sensor in alternating pulses.
Clause 39. The method of any of clauses 33-38, wherein the fluid injector system comprises a first fluid reservoir and a second fluid reservoir for delivering a first fluid and a second fluid, respectively; a first fluid path section in fluid communication with the first fluid reservoir and a second fluid path section in fluid communication with the second fluid reservoir; and first and second proximal sensors and first and second distal sensors, wherein the first fluid path section is associated with the first proximal sensor and the first distal sensor and the second fluid path section is associated with the second proximal sensor and the second distal sensor.
Clause 40. The method of any of clauses 33-39, further comprising inserting a manifold comprising the first fluid path section and the second fluid path section into a manifold housing module, wherein the manifold housing module comprises the first and second proximal sensors and the first and second distal sensors, and wherein the manifold positions the first fluid path section and the second fluid path section to interface with the first and second proximal sensors and the first and second distal sensors, respectively.
Clause 41. The method of any of clauses 33-40, wherein the manifold comprises at least one rib for indexing the manifold within the manifold housing module.
Clause 42. The method of any of clauses 33-41, wherein the emitter and the detector of each of the first proximal sensor and the first distal sensor are located behind associated optical surfaces of the manifold housing module, and wherein the at least one rib prevents the manifold from contacting the associated optical surfaces of the manifold housing module.
Clause 43. The method of any of clauses 33-42, wherein the manifold housing module comprises at least one filter for filtering light emitted from the first proximal sensor and the first distal sensor.
Clause 44. The method of any of clauses 33-43, wherein at least one of the manifold and the manifold housing module includes a lens for concentrating or dispersing light emitted from the first proximal sensor and the first distal sensor.
Clause 45. The method of any of clauses 33-44, wherein the manifold housing module comprises a collimator for collimating light emitted from the first proximal sensor and the first distal sensor.
Clause 46. The method of any of clauses 33-45, further comprising: detecting, with a reference detector of the first proximal sensor or the first distal sensor, a reference light that has not passed through the at least one fluid path section; and comparing the reference light to the light that has passed through the at least one fluid path section to determine fluid content of the at least one fluid path section.
Clause 47. The method of any of clauses 33-46, wherein the emitter of at least one of the first proximal sensor and the first distal sensor is arranged to emit light perpendicular to a fluid flow direction through the at least one fluid path section.
Clause 48. The method of any of clauses 33-47, wherein the emitter of at least one of the first proximal sensor and the first distal sensor is arranged to emit light at an angle of between approximately 30° and approximately 60° relative to a fluid flow direction through the at least one fluid path section.
Clause 49. The method of any of clauses 33-48, further comprising: halting an injection procedure of the fluid injector system in response to determining that the at least one fluid path section contains one or more air bubbles having a total air volume above a predetermined volume.
Clause 50. The method of any of clauses 33-49, further comprising: determining, based on the detected light, that the at least one fluid path section is present between the emitter and the detector of each of the first proximal sensor and the first distal sensor.
Clause 51. The method of any of clauses 33-50, wherein the emitter of at least one of the first proximal sensor and the first distal sensor is configured to emit light on the ultraviolet spectrum.
Clause 52. The method of any of clauses 33-51, wherein the emitter of at least one of the first proximal sensor and the first distal sensor is configured to emit light on the infrared spectrum.
Clause 53. The method of any of clauses 33-52, wherein the emitter of at least one of the first proximal sensor and the first distal sensor is configured to emit light on the visible spectrum.
Clause 54. The method of any of clauses 33-53, wherein an index of refraction of a sidewall of the at least one fluid path section is closer to an index of refraction of water than to an index of refraction of air.
Clause 55. The method of any of clauses 33-54, further comprising: determining a cumulative total volume of air passing through the at least one fluid path section during an injection procedure by adding the volume of the air bubble to a previous cumulative total volume of air.

Further details and advantages of the various examples described in detail herein will become clear upon reviewing the following detailed description of the various examples in conjunction with the accompanying figures.

### BRIEF DESCRIPTION OF THE DRAWINGS

**FIG. 1** is a perspective view of a fluid injector system according to an embodiment of the present disclosure;
**FIG. 2** is a schematic view of the fluid injector system according to an embodiment of the present disclosure;
**FIG. 3** is a front cross-sectional view of a sensor module according to an embodiment of the present disclosure;
**FIG. 4** is a front cross-sectional view of the sensor module of **FIG. 3** associated with a liquid-filled fluid path section;
**FIG. 5** is a front cross-sectional view of the sensor module of **FIG. 3** associated with an air-filled fluid path section;
**FIG. 6** is a top cross-sectional view of the sensor module of **FIG. 3** associated with a liquid-filled fluid path section containing an air bubble;
**FIG. 7** is a top cross-sectional view of a sensor module according to an embodiment of the present disclosure, associated with a liquid-filled fluid path section containing an air bubble;
**FIG. 8** is a perspective view of a manifold and associated sensor module according to an embodiment of the present disclosure;
**FIG.** 9 is a cross-sectional perspective view of the manifold of **FIG. 8** engaged with a manifold housing module including a sensor module according to an embodiment of the present disclosure;
**FIG. 10** **is** a top cross-sectional view of the manifold and manifold housing module of **FIG. 9** including two sensor modules;
**FIG. 11** is a top view of the manifold and manifold housing module of **FIG. 9****;**
**FIG. 12** is a side cross-sectional view of a syringe tip and a sensor module according to an embodiment of the present disclosure;
**FIG. 13** is a schematic of a sensor module according to an embodiment of the present disclosure;
**FIG. 14** is a schematic of a sensor module according to an embodiment of the present disclosure;
**FIG. 15** is a perspective view of a single manifold with a syringe outlet according to an embodiment of the present disclosure;
**FIG. 16** is a front cross-sectional view of an eccentric fluid path section;
**FIG. 17** is a side cross-sectional view of a fluid path section having a draft;
**FIG. 18** is a side cross-sectional view of a fluid path section having a surface finish;
**FIG. 19** is a front cross-sectional view of an out-of-round fluid path section;
**FIG. 20** is a front cross-sectional view of a fluid path section having a wisp;
**FIG. 21** is a graph of sensor output voltage over time for various conditions of a fluid path section associated with the sensor module;
**FIG. 22** is a graph of sensor output voltage over time for various conditions and configurations of a syringe cap or manifold housing module;
**FIGS. 23A** and **23B** are a flow diagram of a method for monitoring fluid flow through a fluid injector system, according to an embodiment of the present disclosure;
**FIG. 24** is a graph of emitter power over time, according to an embodiment of the present disclosure; and
**FIGS. 25** is a graph of detector output voltage over time, according to an embodiment of the present disclosure.

### DETAILED DESCRIPTION

As used herein, the singular form of "a", "an", and "the" include plural referents unless the context clearly dictates otherwise. Spatial or directional terms, such as "left", "right", "inner", "outer", "above", "below", and the like, relate to the invention as shown in the drawing figures and are not to be considered as limiting as the invention can assume various alternative orientations.

All numbers used in the specification and claims are to be understood as being modified in all instances by the term "about". The term "about" is meant to include plus or minus twenty-five percent of the stated value, such as plus or minus ten percent of the stated value. However, this should not be considered as limiting to any analysis of the values under the doctrine of equivalents.

Unless otherwise indicated, all ranges or ratios disclosed herein are to be understood to encompass the beginning and ending values and any and all subranges or sub-ratios subsumed therein. For example, a stated range or ratio of "1 to 10" should be considered to include any and all subranges or sub-ratios between (and inclusive of) the minimum value of 1 and the maximum value of 10; that is, all subranges or sub-ratios beginning with a minimum value of 1 or more and ending with a maximum value of 10 or less. The ranges and/or ratios disclosed herein represent the average values over the specified range and/or ratio.

The terms "first", "second", and the like are not intended to refer to any particular order or chronology, but refer to different conditions, properties, or elements.

All documents referred to herein are "incorporated by reference" in their entirety.

The term "at least" is synonymous with "greater than or equal to". The term "not greater than" is synonymous with "less than or equal to".As used herein, "at least one of" is synonymous with "one or more of". For example, the phrase "at least one of A, B, and C" means any one of A, B, or C, or any combination of any two or more of A, B, or C. For example, "at least one of A, B, and C" includes A alone; or B alone; or C alone; or A and B; or A and C; or B and C; or all of A, B, and C.

The term "includes" is synonymous with "comprises".

When used in relation to a syringe, the term "proximal" refers to a portion of a syringe nearest a fluid injector head for engaging with an end wall of the syringe and delivering fluid from a syringe. When used in relation to a fluid path, the term "proximal" refers to a portion of the fluid path nearest to an injector system when the fluid path is connecting with the injector system. When used in relation to a syringe, the term "distal" refers to a portion of a syringe nearest to a delivery nozzle. When used in relation to a fluid path, the term "distal" refers to a portion of the fluid path nearest to a patient when the fluid path is connected with an injector system. The term "radial" refers to a direction in a cross-sectional plane normal to a longitudinal axis of a syringe extending between proximal and distal ends. The term "circumferential" refers to a direction around an inner or outer surface of a sidewall of a syringe. The term "axial" refers to a direction along a longitudinal axis of the syringe extending between the proximal and distal ends.

It is to be understood that the disclosure may assume alternative variations and step sequences, except where expressly specified to the contrary. It is also to be understood that the specific devices and processes illustrated in the attached drawings, and described in the following specification, are simply exemplary aspects of the disclosure. Hence, specific dimensions and other physical characteristics related to the examples disclosed herein are not to be considered as limiting.

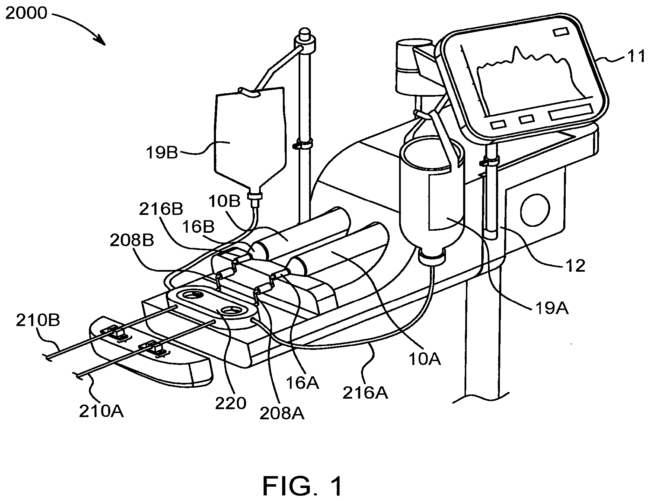

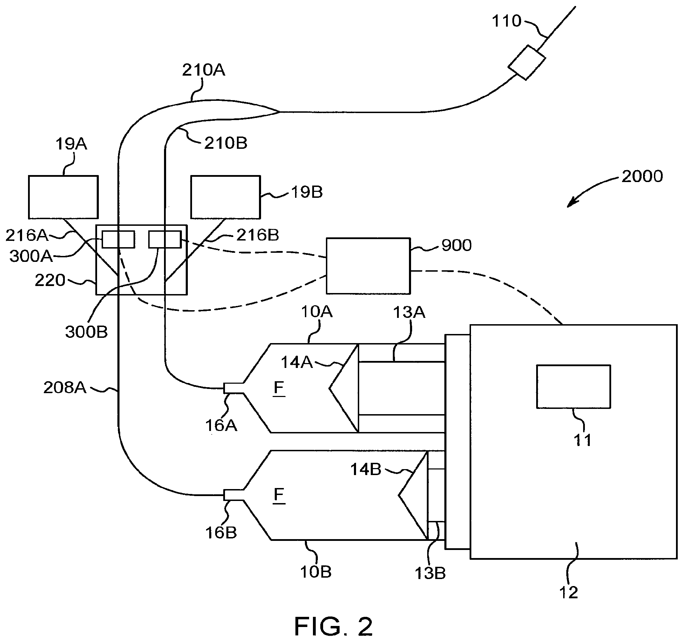

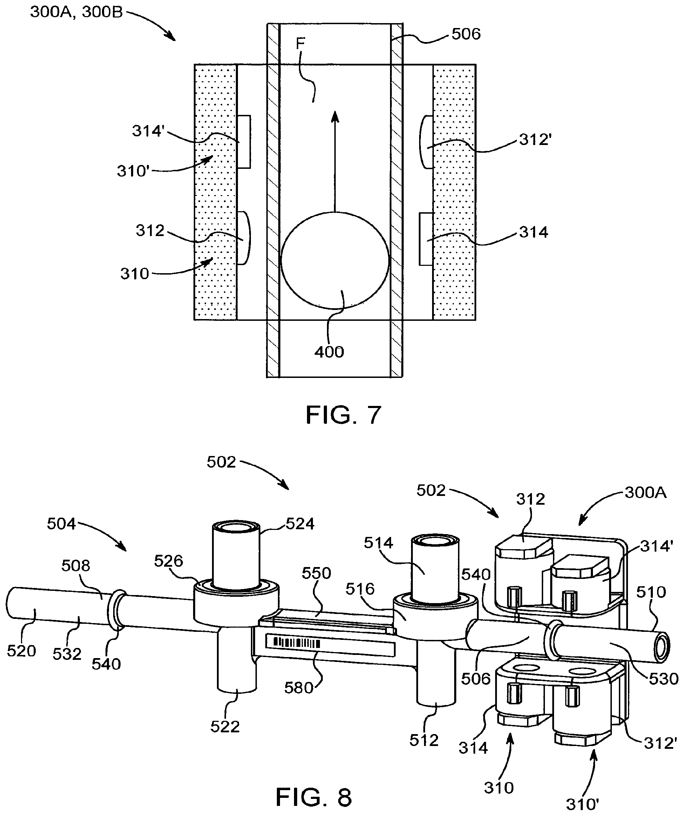

Referring to the drawings in which like reference characters refer to like parts throughout the several views thereof, the present disclosure provides systems, components, devices, and methods for detecting and analyzing fluid content and amount of air of a fluid path section during an injection procedure. Referring first to **FIGS. 1** and **2****,** embodiments of a dual syringe fluid injector system 2000 is illustrated. The fluid injector system 2000 is configured for injection of two medical fluids from respective fluid reservoirs 10A, 10B, which are illustrated as syringes in the accompanying drawings. In some embodiments, the first fluid reservoir 10A contains an imaging contrast media for an angiography, MRI, PET, or computed tomography injection procedure, and the second fluid reservoir 10B contains a flushing fluid, such as saline or Ringer's lactate. The fluids are injected from fluid reservoirs 10A, 10B through a series of fluid path elements connecting the fluid reservoirs 10A, 10B to a catheter 110 inserted into the vasculature system of a patient. The fluid injector system 2000 may further include bulk fluid containers 19A and 19B for filling and refilling the respective syringes 10A, 10B with imaging contrast media and flushing fluid, respectively. The system 2000 includes a fluid path set including a first syringe line 208A in fluid communication with a tip or nozzle 16A of the first syringe 10A, a first fill line 216A in fluid communication with the first bulk fluid container 19A, and a first patient line 210A in fluid communication with the catheter 110. In some embodiments, the first syringe line 208A, the first fill line 216A, and the first patient line 210A are fluidly connected at a manifold 500 (see, e.g., **FIG. 8****)** releasably secured to a manifold housing module 220 of fluid injector 12. The fluid path set further includes a second syringe line 208B in fluid communication with a tip or nozzle 16B of the second syringe 10B, a second fill line 216B in fluid communication with the second bulk fluid container 19B, and a second patient line 210B in fluid communication with the catheter 110. In some embodiments, the second syringe line 208B, the second fill line 216B, and the second patient line 210B are fluidly connected at the manifold 500 **(****FIG. 8****).** The arrangement of the fluid path set allows fluid to be drawn from the first bulk fluid container 19A into the first syringe 10A via the first fill line 216A and the first syringe line 208A. Fluid can be injected from the first syringe 10A to the patient via the first syringe line 208A, the first patient line 210A, and the catheter 110. Similarly, fluid may be drawn from the second bulk fluid container 19B into the second syringe 10B via the first fill line 216B and the first syringe line 208B. Fluid can be injected from the second syringe 10B to the patient via the first syringe line 208B, the first patient line 210B, and the catheter 110. While the fluid injector 12 illustrated in **FIGS. 1** and **2** is shown with a first contrast syringe and a second flushing fluid syringe, in certain injection procedures, only contrast may be used, with no associated flushing fluid. According to these embodiments, the fluid injector 12 may be engaged with only a first syringe 10A and associated first bulk reservoir 19A and fluid path components for injecting the contrast into a patient. The flush side of the fluid injector 12 may be left empty during such a single fluid injection procedure. Alternatively, a fluid injector (not shown) configured for engagement with only a single syringe may utilize the various embodiments of the sensor modules, manifolds, manifold housing modules, and associated air detection and volume determination methods described herein.

Further details and examples of suitable nonlimiting powered injector systems, including syringes, tubing and fluid path components, shut-off valves, pinch valves, and controllers, are described in U.S. Patent Nos. 5,383,858; 7,553,294; 7,666,169; 8,945,051; 10,022,493; and 10,507,319, and International PCT Application Nos. PCT/US2013/061275; PCT/US2018/034613; PCT/US2020/049885; PCT/US2021/035273; PCT/US2021/029963; PCT/US2021/018523; PCT/US2021/037623; PCT/US2021/037574; and PCT/US2021/045298, the disclosures of which are incorporated by reference in their entireties.

With continued reference to **FIGS. 1** and **2****,** the injector system 2000 includes a first piston 13A and second piston 13B respectively associated with each of the syringes 10A, 10B. Each of the pistons 13A, 13B is configured to drive a respective plunger 14A, 14B within a barrel of the respective syringe 10A, 10B. The fluid injector system 2000 includes a controller 900 in electronic communication with various components of the system 2000 to execute an injection procedure, including, for example, monitoring the progress of the injection procedure, tracking the volume of air passing through the fluid path elements, for example by using the various embodiments of the air sensor modules described herein, and, if the volume of air passing through the fluid path elements exceeds a certain threshold volume, stopping the injection procedure so that an amount of air in excess of the threshold volume is not injected into the patient. **In** particular, the controller 900 may include at least one processor programmed or configured to actuate the pistons 13A, 13B and various other components of the injector system 2000 to deliver medical fluids according to a programmed protocol for an injection procedure. The controller 900 may include computer readable media, such as memory, on which one or more injection protocols may be stored for execution by the at least one processor. The controller 900 is configured to actuate the pistons 13A, 13B to reciprocatively move the plungers 14A, 14B within the syringes 10A, 10B and thereby execute and halt an injection procedure. The fluid injector system 2000 may further include at least one graphical user interface (GUI) 11 through which an operator can interact with the controller 900 to view status of and control an injection procedure. In an analogous manner, if the fluid injection system includes one or more pumps, such as a peristaltic pump, the associated controller may operate the various components of the fluid injector, such as the speed of the pumps and the volume of fluid delivered, and monitor and determine the volume of air passing through associated fluid path elements, for example by using the air sensor modules described herein, to ensure the total volume of air passing through the fluid path elements does not exceed a threshold value, and if the total volume of air passing through the fluid path elements exceeds the threshold value, the controller 900 may stop the injection procedure.

The controller 900 may be programmed or configured to execute a filling operation during which the piston 13A, 13B associated with each syringe 10A, 10B is withdrawn toward a proximal end of the syringe 10A, 10B to draw injection fluid F (e.g. imaging contrast media and flushing fluid) into the syringe 10A, 10B from the bulk fluid containers 19A, 19B, respectively. During such a filling operation, the controller 900 may be programmed or configured to selectively actuate various valves, stopcocks, or clamps (such as pinch clamps) to establish fluid communication between the respective syringes 10A, 10B and the bulk fluid containers 19A, 19B via the fill lines 216A and 216B to control filling of the syringes 10A, 10B with the appropriate injection fluid F. According to various embodiments, the fluid may flow through as least a portion of the manifold during the filling operation.

After the filling operation and a priming operation (where excess air is removed from the syringe and various fluid path elements by flowing fluid from the syringe through the fluid path elements), the controller 900 may be programmed or configured to execute a fluid delivery operation during which the piston 13A, 13B associated with one or both of the syringes 10A, 10B is moved toward a distal end of the syringe to inject injection fluid F into the first patient line 210A and the second patient line 210B, respectively, at a specified flow rate and time to deliver a desired amount of fluid F. The controller 900 may be programmed or configured to selectively actuate various valves, stopcocks, and/or pinch clamps to establish fluid communication between the syringes 10A, 10B and the patient, via the patient lines 210A, 210B. The patient lines 210A, 210B ultimately merge before connecting to the catheter 110, for example at a turbulent mixing chamber as described in PCT International Application No. PCT/US2021/019507, the disclosure of which is incorporated herein in its entirety.

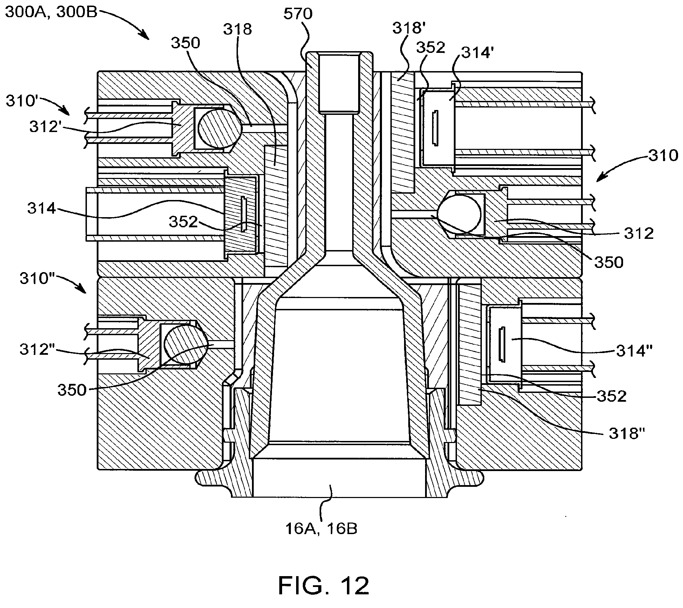

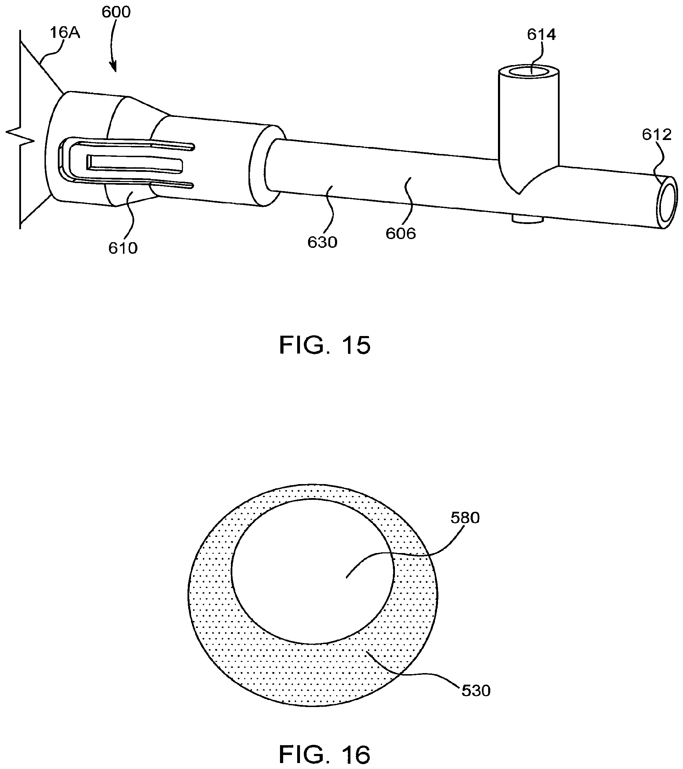

According to various embodiments, the system 2000 includes one or more sensors and/or sensor modules configured for detecting air in the fluid path elements associated with each syringe 10A, 10B. In specific embodiments, the sensor module may include two sensors, a proximal sensor and a distal sensor, arranged linearly along the fluid path element associated with the sensor module. As shown in **FIG.** 2, a first sensor module 300A associated with the first syringe 10A and a second sensor module 300B associated with the second syringe 10B may be located in the manifold housing module 220. The sensor modules 300A, 300B are arranged in operative association with various fluid path sections of the fluid path set. In other embodiments, the sensor modules 300 may be placed at different or additional locations within the system 2000. For example, in the embodiment shown in **FIGS. 12** **and** **15****,** the sensor module 300A, 300B may located at or near respective syringe tips 16A, 16B such that a fluid path section of each of the syringe tips 16A, 16B is in operative communication with the corresponding sensor module 300A, 300B. The sensor modules 300A, 300B are in electronic communication with the controller 900 so that the controller 900 can determine at least one property of a content of the fluid path section based on one or more signals transmitted by the sensor modules 300A, 300B to the controller 900. For example, based on the one or more signals transmitted by the sensor modules 300A, 300B, the controller 900 may be configured to determine an identity of the fluid in the fluid path section, the presence of one or more air bubbles in the fluid path section, a volume of one or more air bubbles in the fluid path section, a velocity of one or more air bubbles in the fluid path section, a total volume of air passing through the fluid path section, a flow rate within the fluid path section, a fluid pressure within the fluid path section, a priming status of the fluid path section, and any combinations thereof.

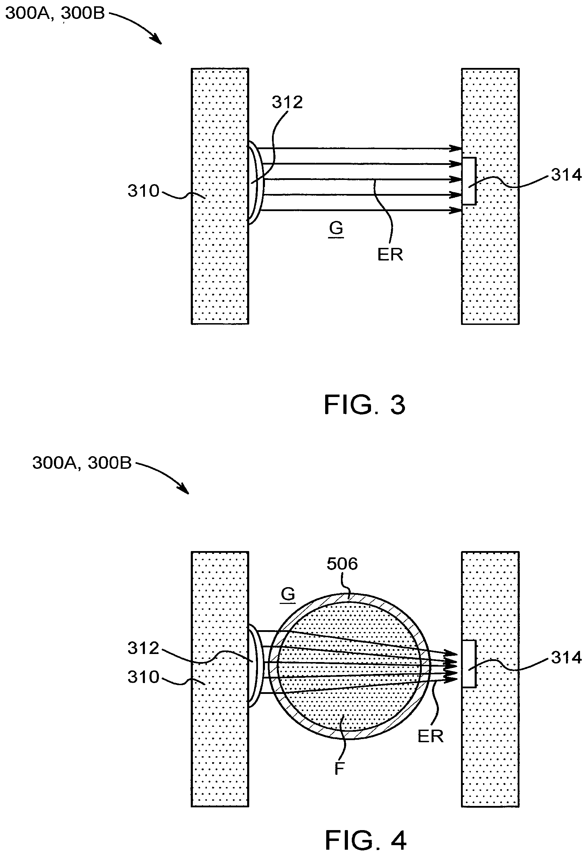

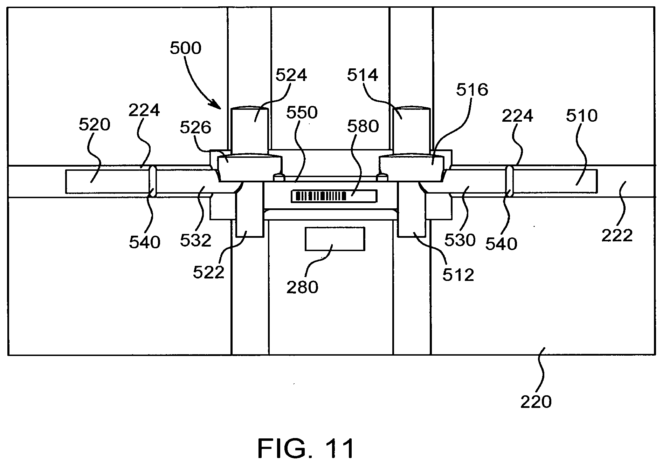

Referring now to **FIGS. 3-5****,** in some embodiments, each sensor module 300A, 300B may include one or more sensors 310 each including an emitter 312 and a collector or detector 314 as illustrated in **FIG. 3****.** The emitter 312 and the detector 314 are spaced apart from one another defining a gap G in which is positioned and operatively associated fluid path section 506, for example a portion of the manifold 500 (see **FIGS. 8-11****)** or the syringe tips 16A, 16B. The emitter 312 is configured to emit electromagnetic radiation ER (e.g. light) at a predetermined wavelength toward the detector 314. The electromagnetic radiation ER must pass through the fluid path section 506 to reach the detector 314. In so doing, the contents of the fluid path section 506 and, in some embodiments, the structure of the fluid path section 506 itself causes the electromagnetic radiation ER to diverge or converge before reaching the detector 314 due to the refraction index of the fluid and the fluid path section 506. Difference in measured refraction may be used to differentiate between an empty sensor 310 compared to one in which the fluid path section 506 has been operatively inserted into the field of the sensor 310. In certain embodiments, the signal from the sensor 310 may further indicate whether the fluid path section 506 has been properly inserted into the sensor 310. Once the fluid path section 506 is correctly installed within the sensor, the sensor may then use differences in measured refraction to determine whether the fluid path second contains a liquid fluid (contrast or aqueous flushing fluid) or air.

In some embodiments, the emitter 312 may be one or more light emitting diodes (LEDs) or liquid crystals configured to emit electromagnetic radiation ER at a predetermined wavelength (or range of wavelengths), although other emitter light sources are within the scope of the present disclosure. In certain embodiments, the emitter 312 may be able to emit electromagnetic radiation ER at more than one wavelength, depending on the fluid to be measured. For example, the emitter 312 may be configured to emit light at a first wavelength and emit light at a second or other wavelength depending on the requirements of the fluid injection procedure. The detector 314 may be any detector capable of converting a quantity of received light into an electrical signal, for example a photodiode or a photodiode array. In various embodiments, the detector 314 may be configured to measure an amount of received electromagnetic radiation ER at different specific wavelengths, depending on the wavelength emitted by the emitter 312. In some embodiments, the emitter 312 is configured to emit electromagnetic radiation on the infrared (IR) spectrum, for example between about 750 nanometers (nm) and about 2000 nm. In some embodiments, the emitter 312 is configured to emit electromagnetic radiation on the ultraviolet (UV) spectrum, for example between about 10 nm and about 400 nm. In some embodiments, the emitter 312 is configured to emit electromagnetic radiation on the visible spectrum, for example between about 380 nm and about 760 nm. In particular embodiments, the electromagnetic radiation emitted by the emitter 312 may have a wavelength from about 1350 nm to about 1550 nm, and in specific embodiments of about 1450 nm. In other embodiments, the electromagnetic radiation emitted by the emitter 312 may have a wavelength within the IR section of the spectrum from about 750 nm to about 950 nm, or in another embodiment from about 800 nm to about 900 nm. In some embodiments, the emitter 312 may be configured to emit acoustic, e.g. ultrasonic, energy, and the detector 314 may be configured to detect acoustic energy. Electromagnetic radiation in the aforementioned wavelengths may have an advantage over other imaging protocols, such as ultrasound, in that electromagnetic radiation does not require acoustic coupling (e.g. compressive contact) between the fluid path section 506 and the sensor 310.

The specific wavelength of electromagnetic radiation may be selected based on the fluids F used in the injection procedure and the structural properties of the fluid path section 506. Particularly, the wavelength(s) of electromagnetic radiation may be chosen that provide maximum differentiation in the output signal of the detector 314 when liquid is present in the fluid path section 506 compared to when air is present in the fluid path section 506. Additionally, the wavelength(s) of electromagnetic radiation may be chosen to minimize adverse effects of factors that can affect sensor performance, such as alignment of the electromagnetic radiation emitter 312 and the detector 314, alignment of the fluid path set 506 with the emitter 312 and the detector 314; the material and geometry of the outer sidewall of the fluid path section 506; and exposure of the detector 314 to ambient light.

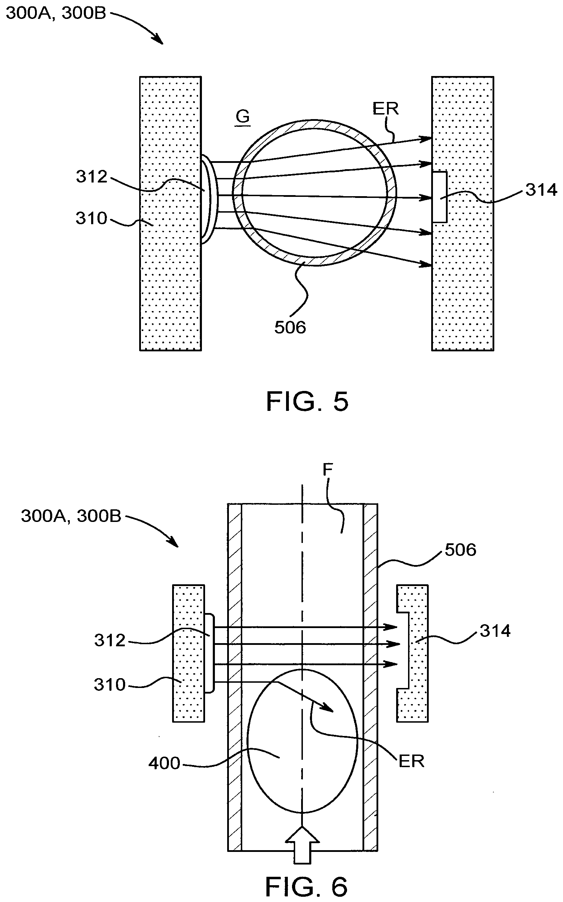

**FIG. 3** illustrates the absence of a fluid path section in the gap G, so the electromagnetic radiation ER must pass through only the air in the gap G to reach the detector 314. **FIG. 4** illustrates the fluid path section 506 placed in the gap G in operative association with the sensor 310. The fluid path section 506 in **FIG. 4** is filled with the injection fluid F as would be expected of a primed fluid path during an injection procedure. The refractory index of the injection fluid F may cause the electromagnetic radiation ER passing through the fluid path section 506 to converge before reaching the detector 314, thereby causing an increase in signal intensity received and measured by the detector 314. **FIG. 5** illustrates the fluid path section 506 placed in the gap G in operative association with the sensor 310, where the fluid path section 506 is at least partially filled with air as would be expected prior to priming the fluid path section 506, or which may occur if an air bubble is present in the injection fluid F during an injection procedure. The refractory index of the air may cause the electromagnetic radiation ER passing through the fluid path section 506 to diverge before reaching the detector 314, thereby causing a decrease in signal intensity receive and measured by the detector 314.

In specific embodiments, light absorption by the content between the emitter 312 and detector 314 may cause a difference in signal intensity measured by the detector 314. For example, in **FIG. 3****,** where no fluid path section 506 is present, the light may pass freely from the emitter 312 to the detector 314 of the sensor 310 with only a minimum of decrease in signal intensity, since air has only a minimal absorption of light from the emitter (which can be factored into any calculation). When a fluid filled fluid path section 506 is inserted into the sensor 310, the signal of light passing from the emitter 312 to the detector 314 is attenuated by absorption by the molecular makeup of the sidewalls of the fluid path as well as the fluid within the fluid path section 506. In conditions where the fluid path section 506 is filled with air or with a mixture of air and medical fluid, for example when a small air bubble is passing therethrough, the signal of light passing from the emitter 312 to the detector 314 is attenuated by absorption by the molecular makeup of the sidewalls of the fluid path section 506 (no absorption by the unprimed air in the fluid path or in a large air bubble), and in the case where both air and fluid are present within a partially filled fluid path section 506 (cross sectional volume of air bubble is less than the cross sectional volume of the fluid path section 506), the signal of light passing from the emitter 312 to the detector 314 is attenuated by absorption by the molecular makeup of the sidewalls of the fluid path section 506 as well as by the partial volume of the fluid within the fluid path section 506. In various embodiments, the detector 314 may be able to use the difference in light attenuation resulting from different liquids within the fluid path to differentiate between different contrast types or concentrations; or between contrast and saline within the fluid path section 506.

**FIG. 6** illustrates a top view of the sensor 310 with an air bubble 400 traveling through the fluid path section 506. As the liquid-air surface interface at the front of the bubble 400 enters the field of the electromagnetic radiation ER generated by the emitter 312, the electromagnetic radiation ER begins to diverge due to the refractory index of the air bubble 400 relative to the refractory index of the injection fluid F and/or to attenuate due to the difference in absorptive properties of the fluid vis-à-vis air. As may be appreciated from **FIG. 6****,** the emitter 312 and the detector 314 may be arranged such that the emitter 312 projects the electromagnetic radiation approximately perpendicular to the fluid flow through the fluid path section 506. As the air bubble 400 continues past the sensor 310, the detector 314 continues to register the reduction in signal intensity until the air-liquid surface interface at the back end of the bubble 400 passes out of the sensing region of the sensor 310. In various embodiments, the air bubble then continues down the fluid path second 506 to the distal sensor 310' (see FIG. 7) where the measurement process is repeated. The signal data from the first proximal sensor and second distal sensor may then be sent to the controller 900 and the controller 900 may calculate various properties of the air and fluid within the fluid path section 506, as described herein.

With continued reference to **FIGS. 3-6****,** the detector 314 is configured to transmit an output signal (e.g. an output voltage) to the controller 900 based on signal strength from the detected electromagnetic radiation ER. Thus, the output signal will be different depending on the refractory index and absorptive properties of the contents in the gap G, allowing the controller 900 to determine whether the fluid path section 506 is absent (as in **FIG. 3****),** the fluid path section 506 is present and filled with medical fluid F **(****FIG. 4****),** or the fluid path section 506 is present and filled at least partially with air **(****FIGS. 5** and **6****).**

Referring now to **FIG. 7****,** each sensor module 300A, 300B may include more than one sensor 310 arranged in series along a flow direction of the injection fluid F. In some embodiments, each sensor module 300A, 300B may include a proximal sensor 310 substantially as described in connection with **FIGS. 3-6****,** and a distal sensor 310' which may be essentially identical in structure to the proximal sensor 310 but located downstream of the proximal sensor 310. In various embodiments, the emitter 312' of the distal sensor 310' may be configured to emit electromagnetic radiation at the same wavelength and/or frequency as the emitter 312 of the proximal sensor 310, or at a different wavelength and/or frequency than the emitter 312 of the proximal sensor 310. In certain embodiments, the distal sensor 310' may be arranged such that the emitter 312' of the distal sensor 310' is arranged on an opposite side of the fluid path section 506 (i.e. approximately 180° about the fluid path section) relative to the emitter 312 of the proximal sensor 310. Likewise, a detector 314' of the distal sensor 310' may be arranged on an opposite side of the fluid path section 506 (i.e. approximately 180° about the fluid path section) relative to the detector 314 of the proximal sensor 310. This arrangement prevents or substantially reduces electromagnetic radiation ER from the emitter 312 of the proximal sensor 310 from being detected by the detector 314' of the distal sensor 310', and electromagnetic radiation ER from the emitter 312' of the distal sensor 310' from being detected by the detector 314 of the proximal sensor 310. In other embodiments, proximal sensor 310 and distal sensor 310' may be arranged at any angle relative to one another.

In other embodiments, the emitters 312, 312' of the proximal and distal sensors 310, 310' may be arranged on the same side of the fluid path section, and the detectors 314, 314' of the proximal and distal sensors 310, 310' may be arranged on the same side of the fluid path section 506. Sufficient space between the sensors 310, 310' and/or optical shields provided between the sensors 310, 310' may be used to prevent interference of the generated electromagnetic radiation between the two sensors 310, 310'. Alternatively, the proximal sensor 310 may use electromagnetic radiation ER having a different wavelength than the distal sensor 310' to avoid cross interference of electromagnetic radiation emitted by the two sensors.

In some embodiments, the emitters 312, 312' of the proximal and distal sensors 310, 310' may be configured to emit electromagnetic radiation in alternating, time-offset, e.g. nonoverlapping, pulses so that there is no confusion as to which emitter 312, 312' is producing electromagnetic radiation at any given time. Additionally, the controller 900 may set time intervals during which neither emitter 312, 312' is producing electromagnetic radiation. The controller 900 can use the signal generated by the detectors 314, 314' during these intervals as a reference for the effect of ambient light on the output signal, and the controller 900 may correct subsequent output signals to account for the effects of ambient light. The sensor modules 300A, 300B may also include filters (as shown in **FIG. 12****)** configured to filter out wavelengths and/or frequencies typical of ambient light.

The implementation of two sensors 310, 310' in series allows the controller 900 to detect velocity and volume of an air bubble 400 in the fluid path section 506 and may calculate the total volume of air at atmospheric pressure based on an applied pressure within the syringe. The velocity of the air bubble 400 may be determined based on a time offset between detection of the air bubble 400 by the proximal sensor 310 and detection of the air bubble 400 by the distal sensor 310'. In some embodiments, the time offset may be calculated from the time the leading edge, liquid-air surface interface of the bubble 400 enters the field of the electromagnetic radiation ER generated by the emitter 312 of the proximal sensor 310 (as shown in **FIG. 6****)** to the time the leading edge of the bubble 400 enters the field of the electromagnetic radiation ER generated by the emitter 312' of the distal sensor 310'. The time at which the leading edge of the bubble 400 is detected by each sensor 310, 310' may be determined by a voltage change in the output of the detectors 314, 314' corresponding to the difference in the refraction index and/or absorption of the air bubble 400 compared to the refraction index and/or absorption of the injection fluid F. In some embodiments, the time offset may be calculated based on the time between the respective detectors 314, 314' detecting the trailing edge of the bubble 400, or based on the time between the respective detectors 314, 314' detecting the largest diameter section of the bubble (associated with the largest change in detector output voltage compared to the liquid-filled fluid path section).

Detection of the flow rate of the air bubble 400 is important because air bubbles may flow faster or slower than the surrounding injection fluid F. In particular, bubbles in the middle of the fluid path section may tend to flow faster than the surrounding injection fluid F, while bubbles on the wall of the fluid path section 506 may flow slower than the surrounding injection fluid F. Additionally, if the fluid path section 506 is oriented such that the fluid flow direction is downward, bubbles may flow slower than the surrounding injection fluid F due to buoyancy influencing the bubbles upward. Thus, the prescribed flow rate of the injection fluid F is not a reliable indicator of the bubble flow rate.

The time offset between the leading edge of the bubble 400 being detected by the sensors 310, 310' may also be used as a component of calculating a flow rate of the air bubble 400. As the bubble continues past the sensors 310, 310', the trailing edge of the bubble is noted once the output signal of the detector 314' falls below the predetermined threshold, indicating that the trailing edge of the air bubble has passed through the detection regions of proximal and distal sensors 310, 310', the controller 900 records the total time for which the output signal of the detector 314, 314' exceeded the predetermined threshold.

In some embodiments, the controller 900 may be configured to calculate the volume of the air bubble 400 based on the flow rate of the air bubble, the total time for which the output signal of the detector 314, 314' exceeded the predetermined threshold, and other known values such as pressure in, and cross-sectional area of, and volume of the fluid path section 506. The volume calculated in this manner will be dependent on the fluid pressure within the fluid path section 506. Thus, to obtain a useful volume measurement, the fluid pressure within the fluid path section 506 must be known or estimated so that the controller 900 can accurately account compression of the bubble under high pressures of CT and/or CT injections relative to the significantly lower pressure atmosphere within the patient's vasculature. Pressure values may be dynamically provided by the controller 900 via a pressure transducer associated with the fluid path set. Additionally, the internal cross-sectional area of the fluid path section 506 may need to be known or estimated to accurately calculate flow rate from the bubble velocity, which in turn can be used to calculate bubble volume.

If the volume of air passing through the sensor module 300A, 300B is larger than a predetermined safe volume, for example greater than about 1.0 milliliters (mL) or other volume determined to be medically acceptable (including 0 mL of air), the controller 900 may automatically halt the injection protocol to prevent air from being injected into the patient. If the volume of air is calculated as less than or equal to the predetermined safe volume, the controller 900 may continue with an injection protocol, optionally with a warning to the user (displayed on the GUI 11, for example) that the calculated volume of air is present in the fluid path set. The controller 900 may then note the volume or air that is less than the predetermined safe volume and keep a running tally of the volume of air that has passed by the sensor module 300A, 300B, adding the volume of subsequent bubbles to the running tally to provide a total volume of air during the injection protocol. In certain procedures, more than one smaller air bubble may pass through the sensor module 300A, 300B during an injection protocol. According to these embodiments, the controller 900 may determine the volume of each air bubble and calculate the total accumulated volume of air that has passed through the sensor modules 300A, 300B by adding the individual volumes of the separate air bubbles. The controller 900 may provide a real-time alert or running total volume of air that has passed through the sensor modules 300A, 300B and may alert the user of the total air volume. For example, in certain embodiments, the controller 900 may display the total air volume value on a display on the GUI 11 to inform the user of the running real-time total. As such, the user will be aware of the total injected volume of air and, depending on the health of patient or other factors, may decide to end an injection protocol early if the total air volume reaches a value deemed unsafe for the specific patient. Alternatively, when the total air volume nears a predetermined unsafe total air volume (e.g. 1.0 mL), the controller 900 may provide an alert to the user that too much air is being injected, or the controller 900 may be configured to automatically stop the injection protocol before the total volume of air in the fluid path set becomes unsafe to the patient.

In some embodiments, the proximal sensor 310 may be configured to emit electromagnetic radiation at a different wavelength and/or frequency than the distal sensor 310'. This allows the respective sensors 310, 310' to be optimized for particular tasks. For example, the emitter 312 of the proximal sensor 310 could have a wavelength and frequency optimized to detect properties and/or defects of the fluid path section 506, which could then be used to normalize or correct the measurement data taken by the distal sensor 310'. The emitter 312' of the distal sensor 310' could have a wavelength and frequency optimized for detecting air in the fluid path section 506. The controller 900 could normalize and/or correct the output signal generated by the detector 314' of the distal sensor 310' using the information obtained from the proximal sensor 310.

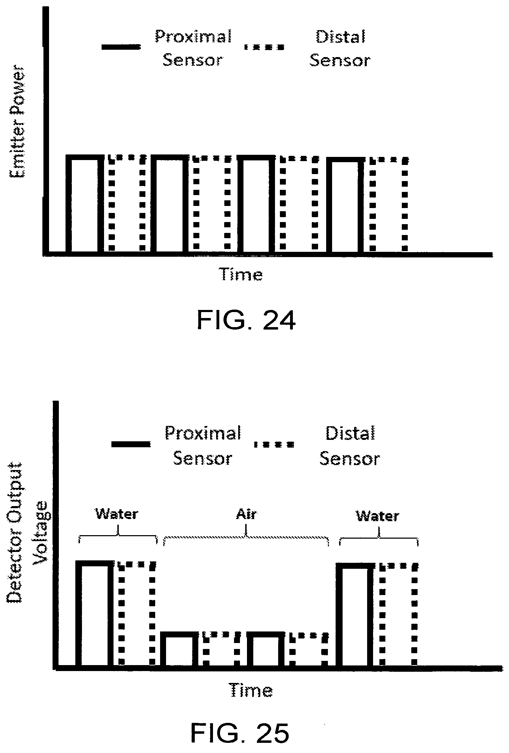

Referring now to **FIG. 24****,** a graph of power supplied to the emitters 312, 312' of the proximal sensor 310 and the distal sensor 310' is shown against time as a bubble passes through the fluid path section 506. As can be appreciated from **FIG. 24****,** emitter power remains constant and unaffected by the presence of the bubble. **FIG. 25** shows a graph of the voltage output of the detectors 314, 314' of the proximal sensor 310 and the distal sensor 310' over the same time interval as the graph of **FIG. 24****.** As can be appreciated from **FIG. 25****,** the voltage output of the detectors 314, 314' decreases when air (e.g. in the form of a bubble) enters the detection range of the sensors 310, 310', as shown by the decrease of the middle bars of the graph under "Air". After the bubble passes the sensors 310, 310', the voltage output of the detectors 314, 314' returns to the original level, as shown in the right-most tow bars of the graph. Thus, while emitter power remains the same, detector output is reduced due the change in refraction and/or absorption of the air bubble relative to the surrounding injection fluid F.

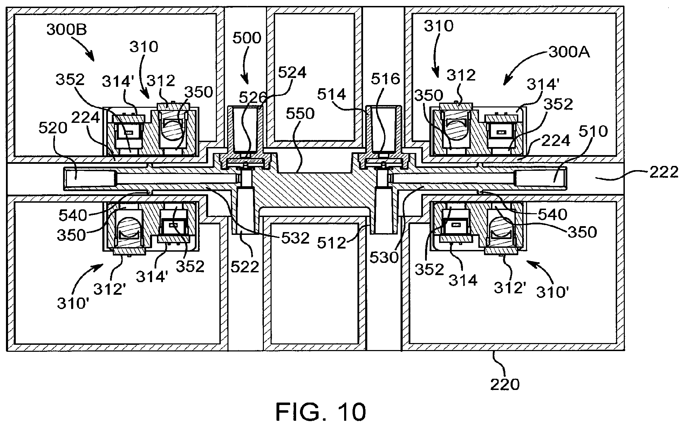

Referring now to **FIG. 8****,** the sensor modules 300A, 300B (sensor module 300B not shown in **FIG. 8****,** see **FIG. 10****)** may be located to operatively interface with the manifold 500 defining the fluid path section to be monitored for air. The manifold 500 includes a first manifold section 502 associated with the first syringe 10A, and a second manifold section 504 associated with the second syringe 10B. The first manifold section 502 defines the first fluid path section 506, which is in fluid communication with a first inlet port 510, a first outlet port 512, and a first fill port 514. The first inlet port 510 is connected to or integrally formed with the syringe line 208A, the first outlet port 512 is connected to or integrally formed with the patient line 210A, and the first fill port 514 is connected to or integrally formed with fill line 216A. Similarly, the second manifold section 504 defines a second fluid path section 508 in fluid communication with a second inlet port 520, a second outlet port 522, and a second fill port 524. The second inlet port 520 is connected to or integrally formed with the syringe line 208B, the second outlet port 522 is connected to or integrally formed with the patient line 210B, and the second fill port 524 is connected to or integrally formed with fill line 216B. The first fluid path section 506 and the second fluid path section 508 are isolated from one another so that imaging contrast flowing through the first fluid path section 506 does not mix with flushing fluid flowing through the second fluid path section 508 and vice versa. The first manifold section 502 and the second manifold 504 may be connected by at least one connecting beam 550. The at least one connecting beam 550 orients and positions the first manifold section 502 and the second manifold section 504 in a position to fit within the manifold housing module 222, and correctly indexes and interfaces the first fluid path section 506 with the sensors 310, 310' of the first sensor module 300A and indexes and interfaces the second fluid path section 508 within the sensors 310, 310' of the second sensor module 300B. Thus, the manifold 500 is designed to allow a user to quickly and accurately install the tubing set into the manifold housing module 220, such that the air detection regions of the fluid flow path are correctly inserted into the reading portions of sensors 310 and 310'. For example, in preparing the fluid injector system 2000 for a new injection procedure, the user may simply connect the syringe lines 208A, 208B to the syringes 10A, 10B, snap the manifold 500 into the manifold housing module 220, and connect the fill lines 216A, 216B to the bulk fluid sources 19A, 19B (for example by spiking the fill lines 216A, 216B into the respective bulk fluid source 19A, 19B) and the fluid path set should be ready for priming. In certain cases, the manifold 500 and the manifold housing module 220 may include complementary latching components, for example on the at least one connecting beam 550, to releasably engage the manifold 500 with the manifold housing module 220. In certain embodiments, the manifold 500 and associated fluid path components may be a disposable component configured for use during a single injection procedure or for a series of injection procedures on a single patient. In other embodiments, the manifold 500 and associated fluid path components may be a disposable component of a multiuse portion of the fluid path set, which can be used in conjunction with multiple single-use portions, over several fluid injection procedures before being disposed of, for example after a set number of injections or 24 hours of use. As noted herein, the manifold 500 described above may be configured for a single fluid injection procedure, e.g., contrast only injection. According to these embodiments, the manifold 500 may only include the first manifold section 502 associated with the first syringe 10A and features designed to index the manifold with sensor 300A. For example, the second manifold section 504 and at least one connecting beam 550 may be molded to releasably engage and fit within the corresponding features of the manifold housing module 220, while indexing the first manifold section 502 with the sensor 300A but may lack the associated fluid path elements in the second manifold section 504, for example, to limit the cost of the single injection fluid injection procedure manifold 500. After use, the manifold 500 and the various fluid lines connected to the manifold 500 are disposed of before use of the fluid injector system 2000 on a subsequent patient.

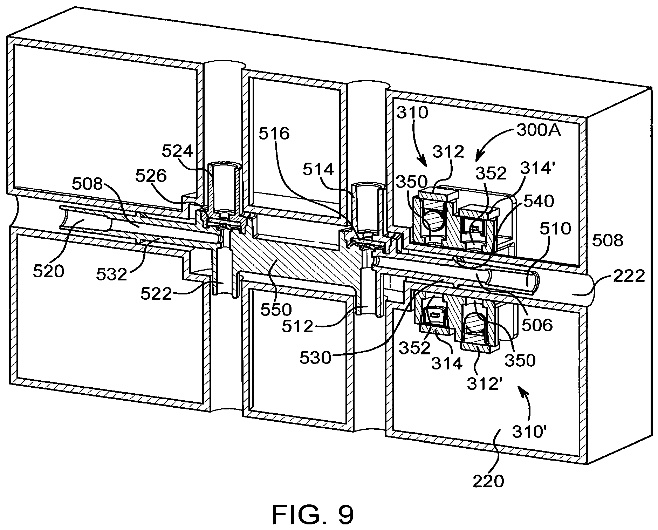

The first fluid path section 506 includes a sidewall 530 configured to allow passage of electromagnetic radiation from the emitters 312, 312' to the detectors 314, 314' when the first fluid path section 506 is disposed in operative association with the sensors 310, 310' of the sensor module 300A. The sidewall 530 is at least partially transparent to the predetermined wavelengths of electromagnetic radiation ER generated by the emitters 312, 312'. The sidewall 530 may be made of an at least partially transparent material, such as a polymer, glass, transparent composite, crystal, or other suitable material. In certain embodiments, the sidewall 530 may be constructed of a plastic material such as polyethylene terephthalate (PET) having a predetermined index of refraction. In some embodiments, the index of refraction of the sidewall 530 is closer to an index of refraction of water than to an index of refraction of air. In some embodiments, the sidewall 530 may be rigid so that the sidewall 530 cannot deflect, which could alter the path of electromagnetic radiation ER through the first fluid path section 506 and cause unreliable sensor readings. In certain embodiments, the sidewall 530 may be curved extending circumferentially around the outer surface of the first fluid path section 506. In other embodiments, the sidewall 530 may have one or more substantially planar exterior surfaces and interior surfaces. The one or more substantially planar surfaces may be located so that the path of electromagnetic radiation from the emitter 312 to the detector 314 passes through the one or more substantially planar surfaces. According to these embodiments, the one or more substantially planar surfaces may minimize or eliminate any focusing or defocusing lensing effect by the surface on the beam of electromagnetic radiation as it passes through the first fluid path section 506. In other embodiments, the sidewall 530 may include or act as a lens to concentrate or disperse the electromagnetic radiation passing through the fluid path section 506. For example, the sidewall 530 may have one or more flat surfaces, which may more predictably transmit light than curved surfaces, and in some embodiments, the sidewall 530 may be a square tube. In some embodiments, the sidewall 530 may have a surface finish to concentrate or disperse the electromagnetic radiation passing through the fluid path section 506. In some embodiments, the sidewall 530 includes one or more ribs 540 extending radially outward from the fluid path section 506. The one or more ribs 540 may be configured to engage the manifold housing module 220, as will be described in connection with **FIGS. 9-****11,** for example to correctly locate the sidewall 530 and the first fluid path section 506 relative to the sensor module 300A and/or prevent contact between the sidewall 530 and a surface of the emitter 312, 312' or the detector 314, 314'.

The second fluid path section 508 includes a sidewall 532 that may be substantially similar to, and may have the same features as, the sidewall 530 of first fluid path section 506.

With continued reference to **FIG. 8****,** the manifold 500 may include one or more check valves, such as check valves 516, 526 respectively located in the fill ports 514, 524. The check valves 516, 526 may act to prevent backflow of fluid into the bulk fluid containers 19A, 19B during a pressurized injection operation. In some embodiments, additional check valves or actively-controlled valves (e.g. stopcocks, pinch valves, etc.) may be located in any of the inlet ports 510, 520, outlet ports 512, 522, and fill ports 514, 524 to selectively control fluid flow through the manifold 500. For example, according to various embodiments, the manifold 500 or manifold housing module 220 may include a check valve or other actively-controlled valve associated with the first fluid path section 506, which may be activated to prevent fluid communication for regions downstream from the first fluid path section 506 with the syringe 10A. According to this embodiment, the valve associated with the first fluid path section 506 may prevent backflow of fluid from the downstream regions back into the syringe 10A during a filling operation where fluid is transferred from the bulk fluid source 19A to the syringe 10A by retraction of the plunger 14A by the piston 13A. Similar features would also be associated with the second fluid path section 508.

With continued reference to **FIG. 8** and further reference to **FIGS. 9-11****,** the manifold 500 may be configured to be inserted into a receiving channel 222 in the manifold housing module 220. In some embodiments, the manifold housing module 220 includes the sensor modules 300A, 300B, and the receiving channel 222 indexes the manifold 500 such that the fluid path sections 506, 508 of manifold 500 are operatively associated with the sensor modules 300A, 300B, respectively. The receiving channel 222 may include optical surfaces 224 behind which the sensors 310, 310' of the sensor modules 300A, 300B are located. The optical surfaces 224 may include or function as lenses for concentrating and/or dispersing the electromagnetic radiation emitted from the emitters 312, 312' and/or detected by detectors 314, 314', as required. The optical surfaces 224 may include or function as a collimator for collimating the electromagnetic radiation emitted from the emitters 312, 312' and/or detected by detectors 314, 314', if required. In addition, the optical surfaces 224 may act to protect the various components of the sensors 310, 310' of the sensor modules 300A, 300B, for example from abrasion or contamination with dirt, dust, contrast agent, or other contaminants which may impact the amount of electromagnetic radiation received by the detectors 314, 314'. In the embodiment shown in **FIGS. 9-11****,** the receiving channel 222 may be arranged such that the portion of the fluid path sections 506, 508 adjacent to the inlet ports 510, 520 are operatively aligned with the respective sensor modules 300A, 300B. As such, the sensor modules 300A, 300B can be used to detect air bubbles flowing into the syringes 10A, 10B via the inlet ports 510, 520 during a filling operation, and to detect air bubbles flowing out of the syringes 10A, 10B via the inlet ports 510, 520 during a fluid injection.

The one or more ribs 540 of the manifold 500 engage the receiving channel 222 of the manifold housing module 220 to index the manifold 500 relative to the sensor modules 300A, 300B. Additionally, the one or more ribs 540 may be located on an outer surface of the first and second fluid path sections 506, 508 to prevent the sidewalls 530, 532 from contacting the optical surfaces 224 of the receiving channel 222 aligned with the sensors 310, 310' to prevent scratching or otherwise degrading the optical properties of the optical surfaces 224 that could adversely affect sensor readings. **In** some embodiments, the receiving channel 222 may include one or more grooves in the manifold housing module 220 to receive the one or more ribs 540 to constrain movement of the manifold 500 within the manifold housing module 220 and index the manifold 500 relative to the manifold housing module 220. In some embodiments, the one or more ribs 540 may instead be provided on the manifold housing block 220 (e.g. extending inward from the receiving channel 222) and the grooves, if provided, may be on the manifold 500. In certain embodiments, the one or more ribs 540 may be located on both the manifold 500 and the manifold housing module 220 and associated grooves may be located on both the respective manifold housing module 220 and manifold 500. In some embodiments, the one or more ribs 540 may be configured to at least partially shield electromagnetic radiation emitted by the emitter 312 of the proximal sensor 310 from being detected by the detector 314' of the distal sensor 310', and to at least partially shield electromagnetic radiation emitted by the emitter 312' of the distal sensor 310' from being detected by the detector 314 of the proximal sensor 310.

As described herein, the manifold 500 and the manifold housing module 220 may include complementary latching components, for example on the at least one connecting beam 550, to releasably engage the manifold 500 with the manifold housing module 220. The controller 900 may be in operative communication with a sensor or detector associated with the latching components, such that the latching components may send a signal to the controller 900 when the manifold 500 is correctly inserted and engaged with the manifold housing module 220. Once the signal that the manifold 500 is correctly engaged is received by the controller 900, the controller 900 may indicate to the user that the system is ready for priming. In other embodiments, when the signal that the manifold 500 is correctly engaged is received by the controller 900, the controller 900 may then automatically begin a priming sequence to prime the fluid path. Alternatively, the controller 900 may ask the user to confirm that the bulk fluid source 19A, 19B has been fluidly connected to the fill lines 216A, 216B and that the syringes 10A, 10B have been fluidly connected to the syringe lines 208A, 208B before initiating the automatic priming sequence. In other embodiments, the manifold 500 may include one or more encoded identifier 580, such as a barcode, QR-code, RFID tag or the like, for example located on the at least one connecting beam 550 or fluid path wall. The fluid injector 12 may have an appropriately positioned reader 280, such as a barcode reader, QR-code reader, RFID reader, associated with the manifold housing module 220. Upon correct engagement of the manifold 500 with the manifold housing module 220, the encoded identifier is read by the reader to determine one or more property of the manifold 500 and associated fluid path elements, such as at least one of: that the manifold 500 is correctly inserted, that the correct manifold 500 for the injection procedure, that the manufacture date of the manifold 500 and associated fluid path components is within the required time frame, and to determine whether the manufacturer of the manifold 500 is an approved manufacturer. If the controller 500 determines that the encoded identifier indicated that there may be an issue with the manifold 500, controller 900 may alert a user and require correction of the issue before the fluid injection procedure may be performed.

With continued reference to **FIGS. 9-10****,** the manifold housing module 220 and/or the sensor modules 300A, 300B may include collimating apertures 350 associated with each of the emitters 312, 312' and/or collimating apertures 352 associated with each of the detectors 314, 314'. The collimating apertures 350 associated with the emitters 312, 312' may restrict the electromagnetic radiation leaving the emitters 312, 312' to a substantially straight trajectory toward the respective detectors 314, 314'. The collimating apertures 352 associated with the detectors 314, 314' may limit the peripheral field of view of the detectors 314, 314' such that only electromagnetic radiation coming from the direction of the respective emitters 312, 312' can reach the detectors 314, 314'. Thus, the collimating apertures 352 may shield the detectors 314, 314' from ambient light sources. In some embodiments, the collimating apertures 350, 352 may have a lesser length than diameter, as shown in **FIGS. 9-11****.** In some embodiments, the collimating apertures 350, 352 may have a greater length than diameter.

In some embodiments, the sensor modules 300A, 300B may be configured to prevent ambient light from effecting detector output signals by pulsing the emitters 312, 312' at a frequency unlike to be present in ambient light sources. For example, the controller 900 and/or the sensor modules 300A, 300B could be configured to pulse the emitters 312, 312' (i.e. rapidly turn the emitters 312, 312' on and off) at a frequency from about 20,000 hertz (Hz) to about 30,000 Hz, and in some embodiments approximately 25,000 Hz. The emitters 314, 314' may be gated so as to ignore electromagnetic radiation not at the same frequency and phase as the pulsing of the emitters 312, 312'. As such, by gating the detectors 314, 314' at approximately 25,000 Hz, the detectors 314, 314' would register the electromagnetic radiation from the emitters 312, 312' being pulsed at approximately 25,000 Hz, but the detectors 314, 314' would ignore sunlight and light from incandescent fixtures (which are not pulsed) and light from fluorescent and LED fixtures (typically pulsed at 50Hz-60Hz AC line frequency).

Referring now to **FIG. 12****,** in some embodiments, the sensor modules 300A, 300B may be operatively associated with the syringe tips 16A, 16B of the syringes 10A, 10B, respectively. The syringe tips 16A, 16B themselves may serve as fluid path sections aligned with the sensors 310, 310', or a separate fluid path section 570 may be attached to the syringe tips 16A, 16B and aligned with the sensors 310, 310'. The fluid path sections 570 in these embodiments may be functionally similar to the fluid path section 506 of the embodiment of **FIGS.** 8-11, having a sidewall that may be at least partially transparent, rigid, and including optical features (e.g. a lens or surface finish) to facilitate use of the sensors 310, 310'. The sensor modules 300A, 300B may be free to rotate about the syringe tips 16A, 16B to allow the operator freedom in positioning the sensor modules 300A, 300B, such as to avoid particular orientations that would receive large amounts of ambient light. Optical filters 318 may be provided between the emitters 312, 312' and the detectors 314, 314' to prevent ambient light from effecting the measurement of the detectors 314, 314'. The optic filters 318 may be configured to block all, or a substantial portion of, wavelengths of electromagnetic radiation greater than and/or less than the wavelength emitted by the emitters 312, 312'. For example, in embodiments in which the emitters 312, 312' are configured to generate electromagnetic radiation at about 1450 nm, the optical filters 318 may be configured to block wavelengths below about 1200 nm and above about 1600 nm.

With continued reference to FIG. 12, the sensor modules 300A, 300B may include one or more additional sensors 310' ' configured to provide further information of the fluid path section 570. An emitter 312' ' of the additional sensor 310'' may be configured to emit electromagnetic radiation ER at the same or a different wavelength than the proximal and distal sensors 310, 310'. In the embodiment shown in FIG. 12, the additional sensor 310'' may be located upstream of the proximal and distal sensors 310, 310'. In other embodiments, the additional sensor 310'' may be located downstream of the proximal and distal sensors 310, 310' or between the proximal and distal sensors 310, 310'. Similar to the embodiment of FIGS. 8-11, the sensor modules 300A, 300B and/or the sidewall of the fluid path section 570 may include complementary ribs and/or grooves to locate of the position the fluid path section 570 relative to the sensor module 300A, 300B. In some embodiments, the conical profile of the syringe tip 16A, 16B may be used to position the fluid path section 570 relative to the sensor module 300A, 300B.

With continued reference to FIG. 12, the sensor modules 300A, 300B may include collimating apertures 350 associated with each of the emitters 312, 312', 312'' and/or collimating apertures 352 associated with each of the detectors 314, 314', 314''. As described in connection with FIGS. 9 and 10, the collimating apertures 350 associated with the emitters 312, 312', 312' ' may restrict the electromagnetic radiation leaving the emitters 312, 312', 312'' to a substantially straight trajectory toward the respective detectors 314, 314', 314''. The collimating apertures 352 associated with the detectors 314, 314', 314'' may limit the peripheral field of view of the detectors 314, 314', 314'' such that only electromagnetic radiation coming from the direction of the respective emitters 312, 312', 312' ' can reach the detectors 314, 314', 314''. In some embodiments, as shown in **FIG. 12****,** the collimating apertures 350 may have a greater length than diameter to increase collimation of electromagnetic radiation from the emitters 312, 312', 312' '.

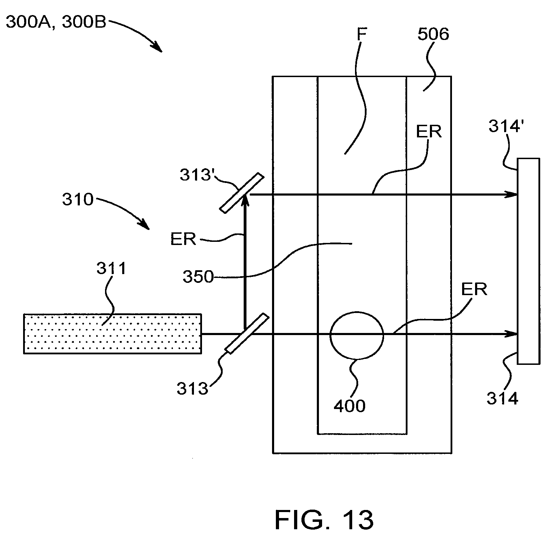

Referring now to **FIG. 13****,** another embodiment of the sensor modules 300A, 300B includes only a single emitter 311 and a pair of reflectors 313, 313' that split electromagnetic radiation ER generated by the emitter 311 into two distinct paths detectable by the proximal and distal detectors 314, 314', respectively. Thus, the embodiment of the sensor modules 300A, 300B of **FIG. 13** can detect the presence of air bubbles at two different locations in the fluid path section 506 like the embodiments of **FIGS. 6-12** with only a single emitter 311. The arrangement of **FIG. 13** may minimize crosstalk that may be associated with having multiple emitters in close proximity; ease alignment of the sensor array using self-calibration and canceling out alignment changes due to lensing; and capture minimum/maximum and set detection thresholds based on detection ranges and system tolerances.

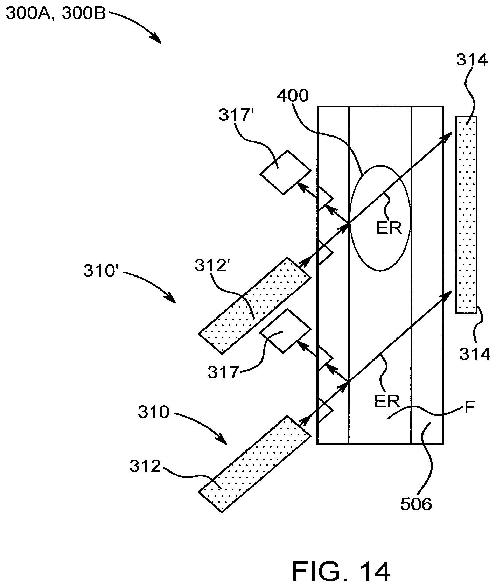

Referring now to **FIG. 14****,** the sensors 310, 310' may be arranged such that the emitters 312, 312' emit electromagnetic radiation ER at an angle other than 90° relative to the fluid path section 506. For example, the emitters 312, 312' and detectors 314, 314' may be arranged such the electromagnetic radiation ER is emitted at an angle between approximately 30° and approximately 60°, in some embodiments approximately 45°, relative to fluid flow through the fluid path section 506. This arrangement increases the distance that the electromagnetic radiation ER must travel to traverse the fluid path section 506, which may increase the sensitivity of the sensors 310, 310'. Further, the angled incident electromagnetic radiation ER may reflect off the surface of the tubing when the fluid path section 506 is empty (filled with air) due to the refractive index difference, and may be detected by reference detectors 317, 317'. This configuration may allow detection of large bubbles with high contrast (at least 4:1), while an empty fluid path (air) reflects significant amount of incident light onto the 45° detector 317, 317' due to a large difference of the index of refraction of the plastic of the fluid path section tubing compared to air. When water or contrast fills the fluid path section 506, the index of refraction of the fluid is closer to that of the sidewall 530 of the fluid path section 506 and reduced reflection and greater transmittance is observed. Thus, according to this embodiment, the angled incident electromagnetic radiation may provide for improved differentiation between air and liquid fluid.

With continued reference to **FIG. 14****,** one or both of the sensors 310, 310' may further include reference detector 317, 317' configured to detect electromagnetic radiation ER reflected off the emitter-side of the fluid path section 506. The reference detectors 317, 317' may be used to calibrate the sensors 310, 310' and provide a baseline measurement of the electromagnetic radiation ER independent of the fluid within the fluid path section 506. The output signals from the reference detectors 317, 317' may be compared to the output signals from detectors 314, 314' to more accurately determine the contents of fluid path section 506.

Referring now to **FIG. 15****,** another embodiment of a manifold 600 includes a rigid, at least partially transparent sidewall 630 with which the sensor modules 300A, 300B (not shown) can be operatively associated, similar to the embodiment shown in **FIGS. 8-11****.** Unlike the embodiment of **FIGS. 8-11****,** the manifold 600 may be configured for attachment to only one of the syringes 10A, 10B, so two manifolds 600, one for each of syringes 10A, 10B, may be used in the system 2000. The manifolds 600 may be used similarly to the separate fluid path sections 570 as discussed with reference to **FIG. 12****.** The manifold 600 and associated sidewall 630 may clip to or otherwise engage with corresponding features of on the tip of syringes 10A, 10B by a clipping engagement mechanism as described in PCT International Application No. PCT/US2021/018523, the disclosure of which is incorporated by this reference in its entirety. The manifold 600 includes an inlet port 610 attached to the syringe tip 16A without intervening flexible tubing. The inlet port 610, the outlet port 612, and the fill port 614 of the manifold 600 may otherwise be substantially the same as the inlet port 510, outlet port 512, and fill port 514 of the manifold 500 of **FIGS. 8-11****.** A fluid path section 606 and associated sidewall 630 in fluid communication with the inlet port 610, the outlet port 612, and the fill port 614 may be positioned in operative association the corresponding sensor module 300A (not shown), and may generally be similar to and include the same features of the fluid path section 506 of the embodiments of **FIGS. 8-14****.**

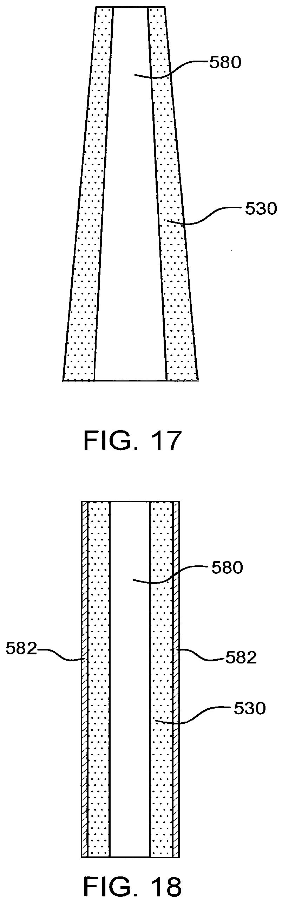

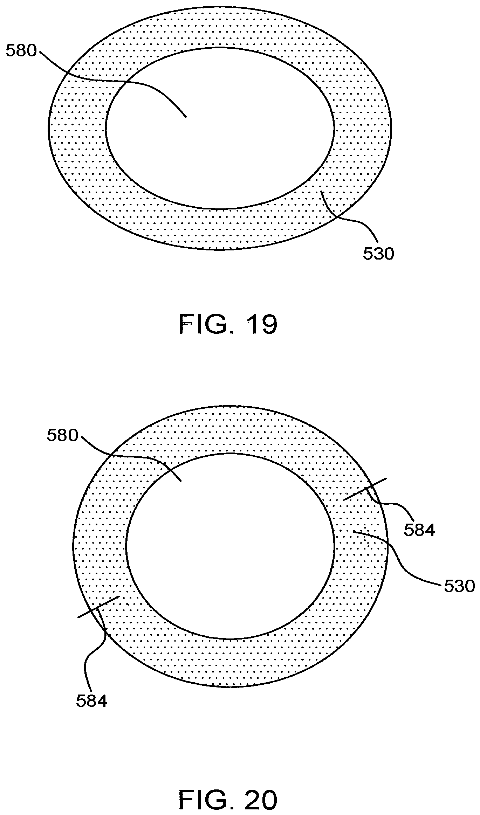

Referring now to **FIGS. 16-20****,** various tubing geometries and manufacturing defects which may be present in the fluid path section associated with the sensors 310, 310' are shown. **FIG. 16** shows an eccentricity in which a lumen 580 of the fluid path section is not concentric with the sidewall 530. **FIG. 17** shows a draft in which the inner diameter and/or outer diameter of the sidewall 530 tapers in a proximal-to-distal direction. **FIG. 18** shows a surface finish 582 applied to the sidewall 530. As described herein, certain surface finishes may be intentional to manipulate the convergence and/or divergence of the electromagnetic radiation passing through the sidewall 530. However, other surface finishes and/or inconsistently applied surface finishes may adversely affect sensor readings and air bubble detection and property identification. **FIG. 19** shows an oval tube in which the inner diameter and/or outer diameter of the sidewall 530 are out of round. **FIG. 20** shows a wisp 584 in the sidewall 530, for example an inclusion in the base material or a molding line imparted during manufacturing. Each of the features shown in **FIGS. 16-20** may cause the electromagnetic radiation passing through the fluid path section to behave in unexpected ways, which can result in spurious and unreliable output signals from the detectors 314, 314'.

In some embodiments, the controller 900 may be configured to perform a test measurement prior to the injection procedure to establish the presence of and potential effects of these geometry features/defects on the output signals from the detectors 314, 314'. The controller 900 may use the results of the test measurement to calibrate the detectors 314, 314' and/or to calculate one or more correction factor based in the effects of the features/defects in one or both the contrast injection fluid paths and the flushing fluid paths. During the injection procedure, the controller 900 may apply the correction factor to the one or more output signals from the detectors 314, 314' and sensor modules 300A, 300B to compensate for the manufacturing feature/defects.

An additional manufacturing issue that can affect sensor readings is the inner diameter of the sidewall 530 being different from an expected value. This can occur due to manufacturing tolerances and/or the use of third party components. An unexpected inner diameter of the sidewall 530 can particularly effect air bubble volume calculations, as the controller 900 may utilize a predetermined diameter constant corresponding to the inner diameter to convert the detected length of the air bubble into a volume. If the actual inner diameter of the sidewall 530 is different than the predetermined diameter constant, the calculation of air bubble volume may be inaccurate. In some embodiments, the controller 900 may be configured to perform a test measurement prior to the injection procedure to establish the sidewall outer diameter, inner diameter, and thickness based on the detected refraction of the empty fluid path section. Based on the test measurement, the controller 900 may apply a correction factor to subsequent output signals from the detectors 314, 314'. In certain embodiments, it may be important that high quality control be exercised during the manufacture of the fluid path components and manifold to prevent measurement errors and, consequently, errors in the volume of air bubbles passing through the detection region and errors in the total volume of air in an injection procedure. As mentioned herein, using correctly manufactured manifold by an approved manufacture may be important for preventing air volume error during fluid injection procedures. Use of an encoded identifier may help prevent inadvertent use of unsuitable fluid path components.

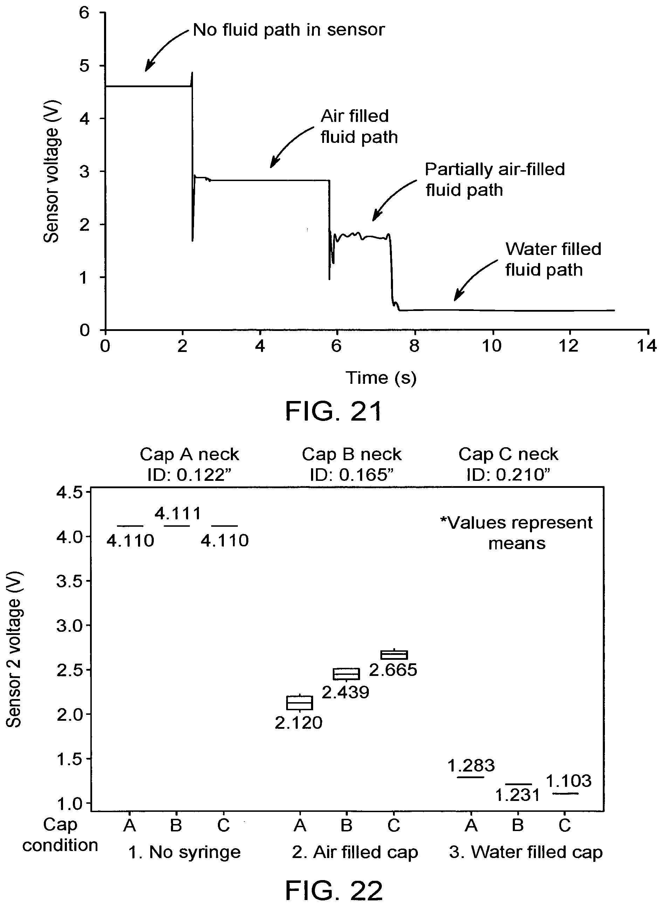

Referring now to **FIG. 21****,** a graph of exemplary output signals of the proximal or distal detector 314, 314' to the controller 900 is shown for emitter 312, 312' operating at 1450 nm. **FIG. 21** illustrates the difference in observed sensor voltage (V) based on the different injector conditions, i.e., no fluid path in sensor, completely air filled fluid path, partially air filled fluid path, and water filled fluid path, and establishes the ability of the controller 900 to discriminate between a condition in which the fluid path section is not positioned in the sensor module 300A, 300B, corresponding to an output signal of between 4 and 5 volts; a condition in which an air-filled fluid path section is positioned in the sensor module 300A, 300B, corresponding to an output signal of approximately 3.0 volts; a condition in which a partially-air-filled fluid path section is positioned in the sensor module 300A, 300B, corresponding to an output signal of approximately 2.0 volts; and a condition in which a water-filled fluid path section is positioned in the sensor module 300A, 300B, corresponding to an output signal of between 0 and 1 volts. As will be understood by one skilled in the art, the illustrated sensor voltage values are for illustration purposes and may vary depending of certain properties, including electromagnetic radiation wavelength or strength, detector configuration, tubing material, diameter, or other property and the like. However, the various embodiments of the sensors 310, 310' and fluid path components according to the present disclosure may accurately differentiate between various conditions associated with the content of the fluid path according to measured values from the sensors 310, 310'.

It is noted that the output signals of the detectors 314, 314' may not respond immediately to changes in the fluid content of the fluid path section, and the change in output signal may exhibit fluctuations or other inconsistent values before reaching a steady state. For example, an air bubble entering the field of electromagnetic radiation of the sensors 310, 310' may initially cause a small drop in the output voltage of the detectors 314, 314', followed by a gradual increase to a steady state output voltage. In some embodiments, the controller 900 may be configured to ignore such fluctuations and inconsistencies before determining that a change in fluid content of the fluid path section has occurred. However, small bubbles flowing through the fluid path section may not occupy the field of electromagnetic radiation of the sensors 310, 310' for along enough time to allow the output signal of the detectors 314, 314' to reach steady state. The controller 900 may be configured to identify such small bubbles by the initial drop in the output voltage signal of the detectors 314, 314', even if the expected steady state output voltage associated with air is never reached. In some embodiments, the controller 900 may be configured to implement a machine learning algorithm to learn the detector output voltage profile associated with a bubble. The controller 900 could then identify the presence of bubbles by identifying this profile in the output signal of the detectors 314, 314'. Additionally, the controller 900 may refine its ability to identify bubbles based on detector output voltage over time using the machine learning algorithm.

Referring now to **FIG. 22****,** a graph of exemplary output signals of the detector 314 is shown for the proximal or distal detector 314, 314' arranged in operative association with syringe tips 16A, 16B (as shown in **FIG. 12****)** of three difference internal diameters (Syringe cap "A" of 0.122 inches, Syringe cap "B" of 0.165 inches, and Syringe cap "C" of 0.210 inches). Tests were performed for each of Syringe Caps "A", "B", and "C" for three different conditions: the syringe cap not in operative association with the sensor module 300A, 300B; the syringe cap in operative association with the sensor module 300A, 300B and filled with air; and the syringe cap in operative association with the sensor module 300A, 300B and filled with water. The output signals from the detector 314 allow the controller 900 to discriminate between these three conditions regardless of the internal diameter of the syringe cap. Across measurements taken for all three syringe cap diameters, the mean output signals for the syringe cap not in operative association with the sensor ranged from 4.110 to 4.111 volts; the mean output signals for the syringe cap filled with air ranged from 2.120 to 2.665 volts; and the mean output signals for the syringe cap filled with water ranged from 1.102 to 1.283 volts. For the test results shown in **FIG. 22****,** the emitter 312 operated at 1450 nm.

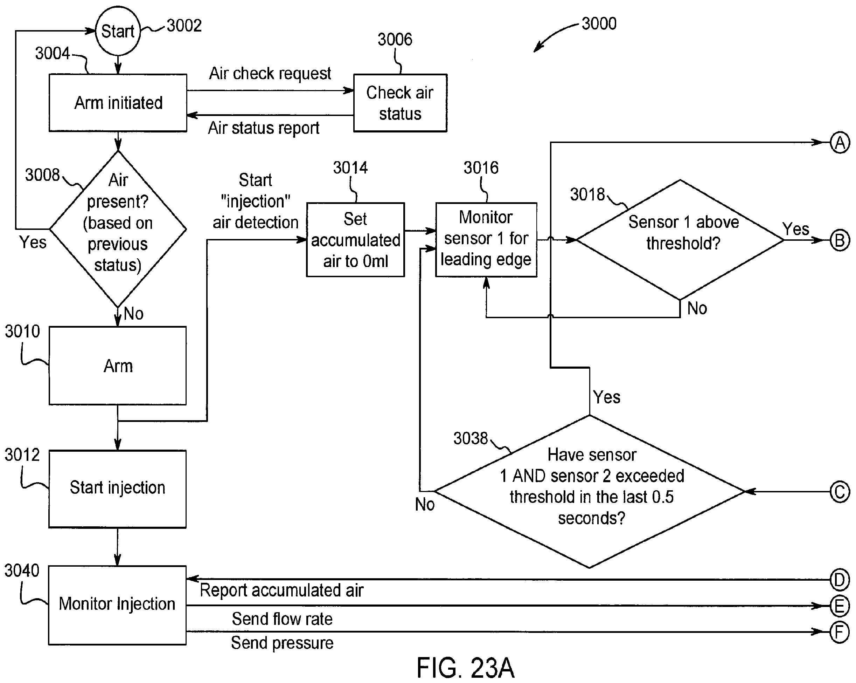

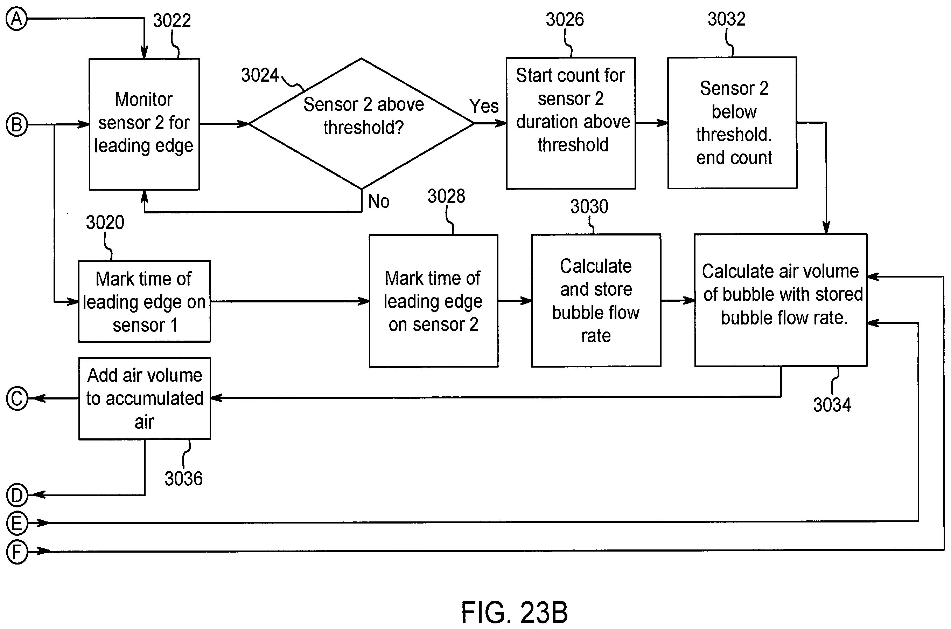

Referring now to **FIGS. 23A** and **23B****,** a flow diagram for a method 3000 for determining one or more fluid properties of a fluid flowing in at least one fluid path section of the fluid injector system 2000 is shown. At step 3002, an injection procedure is started, which may include filling the syringes 10A, 10B from the bulk fluid containers 19A, 19B and priming the fluid path set. At step 3004, the injection procedure is initiated, for example by preloading the pistons 13A, 13B and selectively actuating one or more valves to place the syringes 10A, 10B in fluid communication with the patient. At step 3006, an air check is performed in which the controller 900 determines the presence of air in the syringes 10A, 10B or the fluid path set using procedures and components described herein or in the various patent documents incorporated by reference herein. As step 3008, if air is detected after the priming sequence, the controller 900 may return to step 3002 and proceeds to re-start the injection procedure, which may include alerting the user and re-priming to the system 2000, to purge the detected air. If no air is detected, the controller 900 proceeds to step 3010 and arms the system 2000 for the injection procedure. At step 3012, the injection procedure is started by actuating the pistons 13A, 13B to deliver fluid from the syringes 10A, 10B to the patient at a selected flow rate and a selected volume of each fluid. Concurrently with starting the injection as step 3012, a monitoring procedure is initiated at step 3014 by setting an accumulated air volume total to 0 mL. At step 3016, the controller 900 monitors the proximal sensor 310 for the leading edge of an air bubble in the fluid path section. At step 3018, if the output signal of the detector 314 is below a predetermined threshold, e.g. 0.1 volts, the controller 900 determines that no air is present in the fluid path section and returns to step 3016. If the output signal of the detector 314 is above a predetermined threshold, e.g. 0.1 volts, the controller 900 determines that the leading edge of an air bubble is present and, at step 3020, records the time at which the leading edge of the air bubble was detected by the proximal sensor 310. Then, at step 3022 the controller 900 monitors the distal sensor 310' for the leading edge of the air bubble. At step 3024, if the output signal of the detector 314' is below a predetermined threshold, e.g. 0.1 volts, the controller 900 determines that the air bubble has not reached the distal sensor 310' and returns to step 3022. If the output signal of the detector 314' is above a predetermined threshold, e.g. 0.1 volts, the controller 900 determines that the leading edge of the air bubble has reached the distal sensor 310' and, at step 3026, the controller 900 begins recording the time for which the output signal of the detector' 314 is above the predetermined threshold. Additionally, at step 3028, the controller 900 records the time at which the leading edge of the air bubble was detected by the distal sensor 310'. From these measured values, the flow rate of the air bubble through the detection region may be determined by the controller 900.

At step 3030, the controller 900 calculates the time offset between detection of the leading edge of the air bubble by the proximal sensor 310 and the distal sensor 310', as recorded at steps 3020 and 3028. The controller 900 then calculates the flow rate of the air bubble, as described herein, based on the time offset between detection by the proximal and distal sensors 310, 310'. At step 3032, once the output signal of the detector 314' falls below the predetermined threshold, indicating that the trailing edge of the air bubble has passed through the detection regions of proximal and distal sensors 310, 310', the controller 900 records the total time for which the output signal of the detector 314, 314' exceeded the predetermined threshold. Next, at step 3034, the controller 900 calculates the volume of the air bubble as described herein, based on the flow rate calculated at step 3030, the total time for which the output signal of the detector 314, 314' exceeded the predetermined threshold, and other known values such as pressure in, and cross-sectional area of, and volume of the fluid path section. Pressure values may be dynamically provided by the controller 900 via a pressure transducer associated with the fluid path set. (See step 3040)

At step 3036, the controller 900 adds the air volume calculated at step 3034 to the total accumulated air volume initially set at step 3014. If the total accumulated air exceeds a predetermined safe volume, e.g. 1 mL, the controller 900 may alert the user and/or automatically halt the injection procedure to prevent injection of air in volumes above the predetermined safe volume. At step 3038, the controller 900 determines whether both the proximal and distal sensors 310, 310' have concurrently exceeded the predetermined output signal threshold (e.g. 0.1 volts) for longer than a predetermined time period, e.g. 0.5 seconds. If so, the controller 900 determines that a second air bubble has already entered the detection range of the proximal sensor 310 before the first air bubble has cleared the distal sensor 310'. The controller 900 may assume that the second bubble is travelling at the same velocity as the first bubble, being that the bubbles are in close temporal proximity (e.g. within predetermined time period, e.g. 0.5 seconds, of one another). As such, the controller 900 returns to step 3022 and monitors the distal sensor 310' for the leading edge of the second air bubble. Otherwise, the controller 900 returns to step 3016 and begins monitoring the proximal sensor 310 for the leading edge of subsequent air bubbles.

The injection procedure then continues at step 3040, with continued monitoring by the controller 900. The controller 900 also gathers data using various sensors to use in future iterations of step 3034 - calculating the volume of an air bubble in the fluid path section. For example, the controller 900 may determine pressure in the fluid path section via a pressure transducer associated with the fluid path set.

In some embodiments, the controller 900 may be configured to tally the total volume of air detected at predetermined intervals, e.g. every 200 to 500 milliseconds. This checking can be used to prevent large bubble from reaching the patient, as a bubble may be so large that the controller 900 will not detect a voltage drop indicating that the trailing edge of the air bubble (at step 3032) until the leading edge of the bubble has already reached the patient. To avoid this issue, the check at predetermined intervals ensures the entire bubble need not entire pass the sensors 310, 310' before the controller takes corrective action to halt the injection.

While various examples of the present invention were provided in the foregoing description, those skilled in the art may make modifications and alterations to these examples without departing from the scope and spirit of the disclosure. Accordingly, the foregoing description is intended to be illustrative rather than restrictive. The disclosure described hereinabove is defined by the appended claims, and all changes to the disclosure that fall within the meaning and the range of equivalency of the claims are to be embraced within their scope.

## Claims

1. A fluid manifold for a fluid path component, the fluid manifold comprising:
at least one inlet port configured for fluid communication to at least one fluid reservoir;
at least one outlet port configured for fluid communication to at least one administration line;
at least one fill port configured for fluid communication to at least one bulk fluid source; and
at least one fluid path section in fluid communication with the at least one inlet port, the at least one outlet port, and the at least one fill port, the at least one fluid path section having a sidewall having a predetermined index of refraction such that light passes through the fluid path section at a known refraction.

2. The fluid manifold of claim 1, wherein the index of refraction of the sidewall of the at least one fluid path section is closer to an index of refraction of water than to an index of refraction of air.

3. The fluid manifold of claim 1 or 2, wherein the at least one fluid path section is rigid.

4. The fluid manifold of any of claims 1-3, wherein the at least one fluid path section comprises at least one rib extending radially outward and configured to engage a manifold housing module to index the fluid path section in the manifold housing module.

5. The fluid manifold of any of claims 1-4, wherein the at least one fluid path section has a surface finish configured to concentrate or disperse light passing through the fluid path section, and preferably wherein one of the manifold housing module and the at least one fluid path section comprises at least one lens to concentrate or disperse light passing through the fluid path section.

6. The fluid manifold of any of claims 1-5, wherein the at least one fluid path section is transparent to at least one of ultraviolet light, visible light, and infrared light.

7. The fluid manifold of any of claims 1-6, wherein each of the at least one outlet ports comprises a check valve.

8. The fluid manifold of any of claims 1-7, further comprising:
a first manifold section defining a first fluid path for a first medical fluid;
a second manifold section defining a second fluid path for a second medical fluid; and
at least one connecting beam connecting the first manifold section to the second manifold section,
wherein the first fluid path is isolated from the second fluid path, and
wherein the at least one connecting beam orients the first manifold section and the second manifold section in a position to fit within the manifold housing module and correctly interface the first fluid path with a first proximal sensor and a first distal sensor and interface the second fluid path within a second proximal sensor and a second distal sensor.

9. A method for determining one or more fluid properties of a fluid flowing in at least one fluid path section of a fluid manifold according to any of claims 1 to 8, the method comprising:
emitting light from an emitter of a first proximal sensor through a proximal portion of the at least one fluid path section;
detecting with a detector of the first proximal sensor the light that has passed through the proximal portion of the at least one fluid path section;
emitting light from an emitter of a first distal sensor through a distal portion of the at least one fluid path section;
detecting with a detector of the first distal sensor the light that has passed through the distal portion of the at least one fluid path section; and
determining at least one property of the fluid as it flows through at least one fluid path section based on a difference in light measurement valves determined by the first proximal sensor and the first distal sensor,
wherein the at least one fluid path section has a predetermined index of refraction such that the light passes through the fluid path section at a known refraction.

10. The method of claim 9, wherein determining the at least one property of the fluid comprises determining whether the at least one fluid path section contains a medical fluid, air, or one or more air bubbles.

11. The method of claim 9 or 10, further comprising: determining a velocity of an air bubble passing through the fluid path section based on a time offset between detection of the air bubble by the first proximal sensor and detection of the bubble by the first distal sensor.

12. The method of any of claims 9-11, further comprising: determining a volume of an air bubble passing through the fluid path section based on a time offset between detection of a bubble front and a bubble end of the air bubble by the first proximal sensor and detection of the bubble front and the bubble end of the air bubble by the first distal sensor and a pressure of the fluid within the fluid path section.

13. The method of any of claims 9-12, wherein the first proximal sensor is arranged on a first side of the fluid path section,
wherein the second distal sensor is arranged on a second side of the fluid path section, and
wherein the second side of the fluid path section is approximately 180° opposite the first side of the fluid path section.

14. The method of any of claims 9-13, wherein the fluid injector system comprises a first fluid reservoir and a second fluid reservoir for delivering a first fluid and a second fluid, respectively;
a first fluid path section in fluid communication with the first fluid reservoir and a second fluid path section in fluid communication with the second fluid reservoir; and
first and second proximal sensors and first and second distal sensors, wherein the first fluid path section is associated with the first proximal sensor and the first distal sensor and the second fluid path section is associated with the second proximal sensor and the second distal sensor.

15. The method of claim 14, further comprising inserting the manifold comprising the first fluid path section and the second fluid path section into a manifold housing module,
wherein the manifold housing module comprises the first and second proximal sensors and the first and second distal sensors, and
wherein the manifold positions the first fluid path section and the second fluid path section to interface with the first and second proximal sensors and the first and second distal sensors, respectively.

16. The method of any of claims 9-15, further comprising:
detecting, with a reference detector of the first proximal sensor or the first distal sensor, a reference light that has not passed through the at least one fluid path section; and
comparing the reference light to the light that has passed through the at least one fluid path section to determine fluid content of the at least one fluid path section.

17. The method of any of claims 9-16, further comprising: determining, based on the detected light, that the at least one fluid path section is present between the emitter and the detector of each of the first proximal sensor and the first distal sensor.

18. The method of claim 12, further comprising: determining a cumulative total volume of air passing through the at least one fluid path section during an injection procedure by adding the volume of the air bubble to a previous cumulative total volume of air.
